# EUROPEAN PATENT APPLICATION

(11) **EP 4 120 284 A2**
(43) Date of publication of application: **18.01.2023**
(21) Application number: 22185039.9
(22) Date of filing: 14.07.2022
(51) Int. Cl.: G16H 20/30, A61B 5/00, G16H 30/40, G16H 40/67, A61B 5/11

(54) **IMAGE-BASED RISK ANALYSIS OF INDIVIDUALS IN CLINICAL SETTINGS**

(30) Priority: 15.07.2021 US 202163222222 P; 06.01.2022 US 202263297144 P
(71) Applicant: Hill-Rom Services, Inc., Batesville, IN 47006-9167 (US)
(72) Inventor: KAYSER, Susan A, Batesville, 47006-9167 (US); WOLFE, Gene J, Batesville, 47006-9167 (US); WIGGERMANN, Neal E, Batesville, 47006-9167 (US); RECEVEUR, Timothy J, Batesville, 47006-9167 (US); ZHOU, Jie, Batesville, 47006-9167 (US); BATMAN, Sinan, Batesville, 47006-9167 (US)
(74) Representative: Findlay, Alice Rosemary

(57) **Abstract**

Various techniques for determining risks to patients and care providers based on images are described herein. According to some examples, images of an individual in a clinical setting are identified. A risk that the individual will experience an adverse event, such as an injury, is calculated. An alert or report is output based on the risk.

## Description

This application relates to evaluating and reporting risks of injuries using image-based analyses.

To enhance quality of care and to reduce liability, hospitals and other clinical facilities seek to prevent or minimize injuries to patients under their care. In particular, hospitals take significant efforts to prevent patients from developing hospital acquired pressure injuries (HAPIs). Several scoring systems exist to identify patients at risk of developing a HAPI, notably the Braden Score. However, current assessments for tracking HAPI risks are manually recorded, often inaccurate, and subjective. Moreover, it is impossible for individual care providers to manually assess more complete scoring metrics, due to the number and complexity of HAPI risk factors. Moreover, these scores are typically recorded daily at the beginning of the morning shift by a care provider (e.g., a nurse) caring for multiple patients simultaneously, rather than continuously during the care provider's shift or workday. In addition, communication of these scores or other HAPI risk factors are often incomplete as patient care is handed off from one care provider to another (e.g., during a shift change). The lack of continuous scoring fails to capture changes in the patient condition that could increase or decrease their likelihood of developing a HAPI.

Misidentification of patients at risk leads to over-treatment in some cases and under-treatment in others. Over-treatment can lead to diminished patient satisfaction as patients are subjected to unnecessary and uncomfortable interventions (e.g., patients are turned more frequently than needed, placed on air surfaces unnecessarily which are less comfortable than foam surfaces, etc.). Under-treatment is far more problematic and can lead to the development of HAPIs which can be dangerous for the patient and subject the clinical setting to liability. HAPIs also lead to longer lengths of stay, pain and discomfort, and even death. Other patient risks are similarly difficult to track manually and can have significant consequences to patients, care providers, and clinical settings.

In addition to patient injuries, hospitals also have reason to prevent injuries to care providers. Nursing assistants and registered nurses ranked 2nd and 5th among occupations with the most musculoskeletal injuries (BLS. 2018a. Number, median days away from work, and percentage of total injuries involving musculoskeletal disorders by selected occupations, U.S., private sector, 2018. https://www.bls.gov/iif/oshwc/case/msds-chart3-data.htm). Overexertion injuries are the most common type of injury in these workers. More than half of nurses report having experienced back pain and nearly half report shoulder pain in the previous 12 months (Davis, K.G. and Kotowski, S.E., 2015. Prevalence of musculoskeletal disorders for nurses in hospitals, long-term care facilities, and home health care: a comprehensive review. Human factors, 57(5), pp.754-792).

Hospitals incur substantial direct costs for these injuries through workers compensation insurance premiums and overtime or temporary labor to cover lost shifts. Nurses leaving the profession due to injury or physical stresses also are also a problem as a nursing shortage grows. Nurse turnover costs are estimated at 1.3 times the registered nurse salary (Jones, C.B., 2005. The costs of nurse turnover, part 2: application of the nursing turnover cost calculation methodology. JONA: The Journal of Nursing Administration, 35(1), pp.41-49).

Musculoskeletal injuries are caused by a combination of three factors: high forces, awkward postures, and repetition (or insufficient rest for recovery). High forces and awkward postures are especially common when manually handling patients, and long shifts typical in nursing can prevent adequate recovery of soft tissue which can exacerbate risk of injury. When it comes to limiting risk of injury, there are many guidelines for forces and postures that if followed can protect workers. However, healthcare workers or their managers are often unaware when work tasks exceed these recommended limits and it is not practical for hospitals to perform occupational assessments for every worker. For example, many nurses fail to take actions such as raising the bed to a proper working height when providing care that can reduce risk of injury.

Various implementations of the present disclosure relate to techniques for assessing risks to patients and care providers based on image analysis, as well as outputting warnings based on those risks to prevent or address injuries in a clinical setting. One or more cameras located in a clinical setting obtain images of the patients and care providers over time. A computer-based risk tracking system analyzes the images in order to determine a risk that the patients and care providers will develop injuries. The system, in some cases, outputs various reports and/or alarms to effectively address and/or prevent the injuries from developing.

In particular cases, the system assesses patient risks based on the images. For example, the system may determine a risk that a patient has and/or will develop a pressure injury based on the images. The system may determine the risk of the pressure injury based on a posture of the patient, a frequency of movement of the patient, whether the patient has slid down in a hospital bed, a frequency that the patient is assisted with toileting, and so on. In some cases, the system infers other factors relevant to the pressure injury risk based on the images, such as a weight of the patient, the presence of bony protrusions on the patient, as well as shear forces imposed on the patient's skin. In various implementations, the system determines other types of risks to the patient based on the images, such as a risk that the patient has participated and/or will participate in an unauthorized bed exit, a risk that the patient has and/or will aspirate, a risk that the patient has and/or will experience a fall, a risk that the patient has experienced a seizure, a risk that the patient has been visited by an unauthorized visitor, a risk that the patient is participating and/or will participate in unauthorized eating or drinking, a risk that the patient has absconded and/or will abscond, a risk that the patient has experienced and/or will experience a moment of wakefulness, and a risk that the patient has self-harmed and/or will self-harm. The system may prevent injuries to the patient by notifying a care provider when at least one of these risks has a exceeded a threshold. Based on at least one of the risks, the system outputs an alert on a monitoring terminal used to monitor multiple patients simultaneously.

In some cases, the system determines care provider risks based on the images. For example, the system determines a risk that a care provider has and/or will experience an act of violence by monitoring movements of the care provider and other individuals in the vicinity, such as patients and visitors. In some implementations, the system determines the risk that the care provider will develop an injury while executing ordinary work-based activities, such a risk that the care provider will develop a lifting injury, a posture-based injury, or a repetitive-use injury. In some cases, the system alerts the care provider of the risk in order to prevent the care provider from developing the injury. According to some implementations, the system alerts an administrator of risks of various care providers in the clinical setting, which may enable the administrator to identify common occupational hazards for employees of the clinical setting.

The system may determine and/or confirm any of the risks described herein based on non-image-based data sources, in various examples. For instance, the system may use data obtained from a medical device or a support structure (e.g., a hospital bed) to determine a risk that the patient has and/or will develop an injury. In some cases, the system may track individuals and objects throughout the clinical setting using the images as well as data from a location tracking system monitoring the positions of tags in the clinical setting. The system, in some cases, relies on the electronic medical record (EMR) of the patient to assess the patient's risk for developing an injury. In some examples, the system determines the risk that the care provider will develop an injury based on data from a wearable sensor worn by the care provider. In some implementations, the non-image-based data sources can be used to further enhance the accuracy of determined risks.

Various implementations described herein provide practical improvements to the technical field of automated healthcare management. Clinical settings can rely on care providers to evaluate risks to patients, but with frequent shift changes, multiple care providers caring for each patient at a given time, and incomplete communication of patient statuses between care providers, many patient risks are impossible to manually assess. In contrast, various techniques described herein enable accurate assessment of patient risks and real-time alerts to care providers and other individuals who are capable of addressing those risks. Techniques described herein reduce the burden on care providers and also improve patient outcomes in clinical settings.

The invention will now be further described by way of example with reference to the accompanying drawings, in which:
FIG. 1 illustrates an example environment for tracking individuals in a clinical setting.
FIGS. 2A and 2B illustrate example images of a patient at risk for developing an injury
FIGS. 3A and 3B illustrate additional example images of a patient at risk for developing an injury.
FIGS. 4A to 4C illustrate images of a care provider lifting a moveable subject.
FIGS. 5A and 5B illustrate images of a care provider that is at risk for developing a posture-based injury.
FIGS. 6A and 6B illustrate images of a care provider that is at risk for developing a repetitive-use injury.
FIG. 7 illustrates an example of a user interface that is output by a monitoring terminal.
FIG. 8 illustrates an example process for image-based analysis of the risk of an individual in a clinical setting.
FIG. 9 illustrates an example process for confirming an image-based analysis of the risk of an individual in a clinical setting using supplemental data sources.
FIG. 10 illustrates at least one example device configured to enable and/or perform the some or all of the functionality discussed herein.

Various implementations of the present disclosure will be described in detail with reference to the drawings, wherein like reference numerals present like parts and assemblies throughout the several views. Additionally, any samples set forth in this specification are not intended to be limiting and merely set forth some of the many possible implementations.

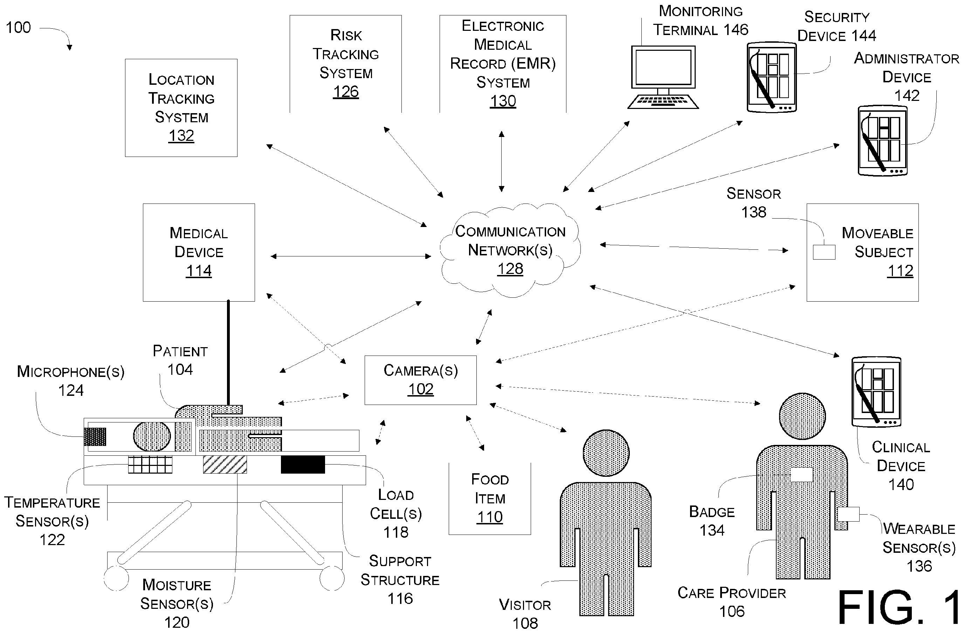

FIG. 1 illustrates an example environment 100 for tracking individuals in a clinical setting. The environment 100 includes one or more cameras 102 configured to capture images of a sub-environment within the environment 100. For example, the camera(s) 102 may be configured to capture images of a room, a curtained area, a hallway, an operating room, or another type of sub-environment within the clinical setting. The clinical setting, for example, may be a hospital, medical clinic, hospice, or any other type of managed care facility.

The images captured by the camera(s) 102 may include one or more objects. As used herein, the term "object," and its equivalents, may refer to a portion of an image that can be interpreted as a single unit and which depicts a single subject depicted in the image. Examples of subjects include machines, devices, equipment, as well as individuals depicted in the images. In some implementations, an object is a portion of a subject that is depicted in the images. For example, a head, a trunk, or a limb of an individual depicted in the images can be an object.

In various implementations, the camera(s) 102 may be monitoring a patient 104 within the clinical setting. In some implementations, the patient 104 is assigned to, or otherwise associated with, the sub-environment monitored by the camera(s) 102. For example, the patient 104 may at least temporarily reside in a room captured by the camera(s) 102. In various cases, at least some of the images captured by the camera(s) 102 may depict the patient 104.

In some cases, the camera(s) 102 may be monitoring a care provider 106 within the clinical setting. The care provider 106, for example, may be a nurse, a nursing assistant, a physician, a physician's assistant, a physical therapist, or some other authorized healthcare provider. In various implementations, at least some of the images captured by the camera(s) 102 may depict the care provider 106.

Further, according to some examples, the camera(s) 102 may be monitoring a visitor 108 within the clinical setting. For example, the visitor 108 may be a friend or family member of the patient 104. The visitor 108 may be authorized or unauthorized. As used herein, the term "unauthorized visitor" can refer to a visitor to a patient who is not allowed to have visitors in a clinical setting. In some cases, at least some of the images captured by the camera(s) may depict the visitor 108.

The images captured by the camera(s) 102 may further include objects depicting other types of subjects. For example, at least some of the images may depict a food item 110. As used herein, the term "food item," and its equivalents, may refer to an orally consumable item and/or a container of an orally consumable item. In some cases, the food item 110 includes a tray, such as a tray used in the clinical setting to carry food and drinks throughout the clinical setting. In some examples, the food item 110 includes a can or cup configured to hold a drink.

In some cases, at least some of the images captured by the camera(s) 102 depict a movable object 112. The movable object 112, in various cases, is physically portable. For example, the movable object 112 may be carried and/or lifted by the care provider 106. Examples of the movable object 112 include medical equipment (e.g., a lead apron), a medical instrument (e.g., a scalpel), or a medical device (e.g., a portable medical imaging device, such as a portable ultrasound imaging device).

In some cases, at least some of the images captured by the camera(s) 102 depict a medical device 114. The medical device 114 may include one or more sensors configured to detect one or more vital signs of the patient 104. As used herein, the term "vital sign," and its equivalents, may refer to a physiological parameter of an individual. Examples of vital signs include pulse or heart rate, temperature, respiration rate, blood pressure, blood oxygenation (e.g., SpO₂), and exhaled CO₂ (e.g., a capnograph). In some examples, the medical device 114 is an assistive lifting device configured to assist with patient ambulation.

Further, according to some implementations, at least some of the images captured by the camera(s) 102 may depict a support structure 116. The support structure 116, for example, may be a hospital bed, a gurney, a chair, or any other structure configure to at least partially support a weight of the patient 104. As used herein, the terms "bed," "hospital bed," and their equivalents, can refer to a padded surface configured to support a patient for an extended period of time (e.g., hours, days, weeks, or some other time period). The patient 104 may be laying down on the support structure 116. For example, the patient 104 may be resting on the support structure 116 for at least one hour, at least one day, at least one week, or some other time period. In various examples, the patient 104 and the support structure 116 may be located in the room. In some implementations, the support structure 116 includes a mechanical component that can change the angle at which the patient 104 is disposed. In some cases, the support structure 116 includes padding to distribute the weight of the patient 104 on the support structure 116. According to various implementations, the support structure 116 can include vital sign monitors configured to output alarms or otherwise communicate vital signs of the patient 104 to external observers (e.g., care providers, visitors, and the like). The support structure 116 may include railings that prevent the patient 104 from sliding off of a resting surface of the support structure 116. The railings may be adjustable, in some cases.

In various examples, the support structure 116 includes one or more sensors. For instance, the support structure 116 may include one or more load cells 118. The load cell(s) 118 may be configured to detect a pressure on the support structure 116. In various cases, the load cell(s) 118 can include one or more strain gauges, one or more piezoelectric load cells, a capacitive load cell, an optical load cell, any device configured to output a signal indicative of an amount of pressure applied to the device, or a combination thereof. For example, the load cell(s) 118 may detect a pressure (e.g., weight) of the patient 104 on the support structure 116. In some cases, the support structure 116 includes multiple load cells that respectively detect different pressures on the support structure 116 in different positions along the support structure 116. In some instances, the support structure 116 includes four load cells arranged at four corners of a resting surface of the support structure 116, which respectively measure the pressure of the patient 104 on the support structure 116 at the four corners of the support structure 116. The resting surface, for instance, can be a surface in which the patient 104 contacts the support structure 116, such as a top surface of the support structure 116.

The support structure 116 may include one or moisture sensors 120. The moisture sensor(s) 120 may be configured to measure a moisture on a surface (e.g., the resting surface) of the support structure 116. For example, the moisture sensor(s) 120 can include one or more capacitance sensors, one or more resistance sensors, one or more thermal conduction sensors, or a combination thereof. In some cases, the moisture sensor(s) 120 include one or more fiber sheets configured to propagate moisture to the moisture sensor(s) 120. In some cases, the moisture sensor(s) 120 can detect the presence or absence of moisture (e.g., sweat or other bodily fluids) disposed between the support structure 116 and the patient 104. For example, the moisture sensor(s) 120 may detect a relative difference in inlet vs. outlet moisture of the support structure 116.

In various examples, the support structure 116 can include one or more temperature sensors 122. The temperature sensor(s) 122 may be configured to detect a temperature of at least one of the patient 104, the support structure 116, or the room. In some cases, the temperature sensor(s) 122 includes one or more thermistors, one or more thermocouples, one or more resistance thermometers, one or more Peltier sensors, or a combination thereof. In particular examples, the temperature sensor(s) 122 detect a relative difference in inlet vs. outlet temperature of the support structure 116.

The support structure 116 may include one or more cameras. For instance, the camera(s) 102 may be part of the support structure 116. The camera(s) may be configured to capture images of the patient 104, the support structure 116, the room, or a combination thereof. In various cases, the camera(s) may include radar sensors, infrared cameras, visible light cameras, depth-sensing cameras, or any combination thereof. In some examples, infrared images may indicate, for instance, a temperature profile of the patient 104 and/or the support structure 116. Thus, the camera(s) may be a type of temperature sensor. In addition, the images may indicate a position of the patient 104 and/or the support structure 116, even in low-visible-light conditions. For example, the infrared images may capture a position of the patient 104 during a night environment without ambient lighting in the vicinity of the patient 104 and/or the support structure 116. In some cases, the camera(s) may include one or more infrared video cameras. The camera(s) may include at least one depth-sensing camera configured to generate a volumetric image of the patient 104, the support structure 116, and the ambient environment. According to various implementations, the images and/or videos captured by the camera(s) are indicative of a position and/or a movement of the patient 104 over time.

According to some examples, the support structure 116 can include one or more video cameras. The video camera(s) may be configured to capture videos of the patient 104, the support structure 116, the room, an entrance to the room, an entrance to a bathroom adjacent to the room, or a combination thereof. The videos may include multiple images of the patient 104 and/or the support structure 116. Thus, the videos captured by the video camera(s) may be indicative of a position and/or movement of the patient 104 over time. In some examples, the video camera(s) capture visible light videos, changes in radar signals over time, infrared videos, or any combination thereof.

In some examples, the support structure 116 can include one or more microphones 124 configured to capture audio signals output by the patient 104, the support structure 116, and/or the ambient environment. The audio signals captured by the microphone(s) 124 may be indicative of a position and/or movement of the patient 104 over time. In particular cases, the microphone(s) 124 are integrated within the camera(s) and/or video camera(s).

In some examples, the support structure 116 includes a head rail and a foot rail. The camera(s) and/or video camera(s), for instance, are mounted on the head rail, the foot rail, an extension (e.g., a metal or polymer structure) attached to the head rail or the foot rail, or any combination thereof. In various implementations, the camera(s) and/or video camera(s) are attached to a wall or ceiling of the room containing the support structure 116. In some examples, the camera(s) and/or video camera(s) are attached to a cart or other object that is located in the vicinity of the support structure 116. In some implementations, the camera(s) and/or video camera(s) are integrated with the electronic whiteboard 102.

In various cases, the sensors (e.g., the load cell(s) 118, the moisture sensor(s) 120, the temperature sensor(s) 122 , the camera(s), the video camera(s), the microphone, or any combination thereof) of the support structure 116 are configured to monitor one or more parameters of the patient 104 and to generate sensor data associated with the patient 104. In various cases, the sensors convert analog signals (e.g., pressure, moisture, temperature, light, electric signals, sound waves, or any combination thereof) into digital data that is indicative of one or more parameters of the patient 104. As used herein, the terms "parameter," "patient parameter," and their equivalents, can refer to a condition of an individual and/or the surrounding environment. In this disclosure, a parameter of the patient 104 can refer to a position of the patient 104, a movement of the patient 104 over time (e.g., mobilization of the patient 104 on and off of the support structure 116), a pressure between the patient 104 and an external object (e.g., the support structure 116), a moisture level between the patient 104 and the support structure 116, a temperature of the patient 104, a vital sign of the patient 104, a nutrition level of the patient 104, a medication administered and/or prescribed to the patient 104, a previous condition of the patient 104 (e.g., the patient was monitored in an ICU, in dialysis, presented in an emergency department waiting room, etc.), circulation of the patient 104 (e.g., restricted blood flow), a pain level of the patient 104, the presence of implantable or semi-implantable devices (e.g., ports, tubes, catheters, other devices, etc.) in contact with the patient 104, a sound emitted by the patient 104, or any combination thereof. In various examples, the load cell(s) 118, the moisture sensor(s) 120, the temperature sensor(s) 122, the cameras, the video camera(s), the microphone(s) 124, or a combination thereof, generates sensor data indicative of one or more parameters of the patient 104.

In various implementations, a risk tracking system 126 is configured to assess a risk of an injury or adverse event of the patient 104 and/or the care provider 106, based on the images captured by the camera(s) 102. The risk tracking system 126 may be implemented in software, hardware, or a combination thereof. For instance, the risk tracking system 126 may be implemented and/or executed by one or more computing devices (e.g., servers) that are located on the premises of the clinical setting and/or outside of the clinical setting. In some cases, the risk tracking system 126 is integrated with the camera(s) 102.

According to various examples, the risk tracking system 126 is connected to one or more communication networks 128. As used herein, the term "communication network," and its equivalents, may refer to at least one device and/or at least one interface over which data can be transmitted between endpoints. For instance, the communication network(s) 128 may represent one or more communication interfaces traversing the communication network(s). Examples of communication networks include at least one wired interface (e.g., an ethernet interface, an optical cable interface, etc.) and/or at least one wireless interface (e.g., a BLUETOOTH interface, a WI-FI interface, a near-field communication (NFC) interface, a Long Term Evolution (LTE) interface, a New Radio (NR) interface, etc.). In some cases, data or other signals are transmitted between elements of FIG. 1 over a wide area network (WAN), such as the Internet. In some cases, the data include one or more data packets (e.g., Internet Protocol (IP) data packets), datagrams, or a combination thereof.

In some cases, the camera(s) 102 generate data indicative of the images and transmit the data to the risk tracking system 126 over the communication network(s) 128. Accordingly, the risk tracking system 126 may analyze the images in order to identify a risk to the patient 104 and/or a risk to the care provider 106.

In various implementations, the risk tracking system 126 tracks one or more objects in the images captured by the camera(s) 102. For example, the risk tracking system 126 detects an object in one of the images. In some implementations, the risk tracking system 126 detects the object using edge detection. The risk tracking system 126, for example, detects one or more discontinuities in brightness within the image. The one or more discontinuities may correspond to one or more edges of a discrete object in the image. To detect the edge(s) of the object, the risk tracking system 126 may utilize one or more edge detection techniques, such as the Sobel method, the Canny method, the Prewitt method, the Roberts method, or a fuzzy logic method.

According to some examples, the risk tracking system 126 identifies the detected object. For example, the risk tracking system 126 performs image-based object recognition on the detected object. In some examples, the risk tracking system 126 uses a non-neural approach to identify the detected object, such as the Viola-Jones object detection framework (e.g., based on Haar features), a scale-invariant feature transform (SIFT), or a histogram of oriented gradients (HOG) features. In various implementations, the risk-tracking system 126 uses a neural-network-based approach to identify the detected object, such as using a region proposal technique (e.g., R convolutional neural network (R-CNN) or fast R-CNN), a single shot multibox detector (SSD), a you only look once (YOLO) technique, a single-shot refinement neural network (RefineDet) technique, a retina-net, or a deformable convolutional network. In various examples, the risk tracking system 126 determines that the detected object is the patient 104, the care provider 106, the visitor 106, the moveable subject 112, or the medical device 114.

In various implementations, the risk tracking system 126 tracks the object throughout the multiple images captured by the camera(s) 102. The risk tracking system 126 may associate the object depicted in consecutive images captured by the camera(s) 102. In various cases, the object is representative of a 3-dimensional (3D) subject within the clinical setting, which can be translated within the clinical setting in 3 dimensions (e.g., an x-dimension, a y-dimension, and a z-dimension). The risk tracking system 126 may infer that the subject has moved closer to, or farther away, from the camera(s) 102 by determining that the object representing the subject has changed size in consecutive images. In various implementations, the risk tracking system 126 may infer that the subject has moved in a direction that is parallel to a sensing face of the camera(s) 102 by determining that the object representing the subject has changed position along the width or height dimensions of the images captured by the camera(s) 102.

Further, because the subject is a 3D subject, the risk tracking system 126 may also determine if the subject has changed shape and/or orientation with respect to the camera(s) 102. For example, the risk tracking system 126 may determine if the visitor 108 has bent down and/or turned around within the clinical setting. In various implementations, the risk tracking system 126 utilized affine transformation and/or homography to track the object throughout multiple images captured by the camera(s) 102.

According to some instances, the risk tracking system 126 identifies the position and/or movement of various subjects depicted in the images captured by the camera(s) 102 by tracking the objects representing those subjects. In various cases, the risk tracking system 126 determines a relative position (e.g., distance) and/or movement of multiple subjects depicted in the images. For example, the risk tracking system 126 is configured to determine whether the patient 104 is touching, moving toward, or moving away, from the support structure 116 using the image tracking techniques described herein.

In various implementations the risk tracking system 126 detects, identifies, and/or tracks objects by recognizing fiducial markers depicted in the images captured by the camera(s) 102. For example, fiducial markers may be affixed to the patient 104, the care provider 106, the visitor 108, the moveable subject 112, the medical device 114, the support structure 116, a door and/or doorframe of the room monitored by the camera(s) 102, or any combination thereof. Examples of fiducial markers include stickers and/or wearable bands (e.g., bracelets) that display a predetermined pattern that is discernible to the risk tracking system 126 when depicted in the images captured by the camera(s) 102. For instance, the fiducial markers are stickers displaying predetermined barcodes (e.g., predetermined quick response (QR) codes). In some cases, the barcodes uniquely identify the subject on which they are affixed. For example, a first sticker with a first QR code is affixed to a right rail of the support structure 116 and a second sticker with a second QR code is affixed to a left rail of the support structure 116. The risk tracking system 126 detects the first QR code and the second QR code in an image captured by the camera(s) 102. In addition, the risk tracking system 126 accesses a look-up table stored in the risk tracking system 126 that correlates the first QR code to the right rail of the support structure 116 and the second QR code to the second rail of the support structure 116. Accordingly, the risk tracking system 126 may identify the support structure 116 and may further identify the orientation of the support structure 116 within the room, based on identifying the fiducial markers depicted in the images.

In various implementations, when the subject is an individual, the risk tracking system 126 further identifies a pose of the individual depicted in the images captured by the camera(s) 102. As used herein, the term "pose," and its equivalents, refers to a relative orientation of limbs, a trunk, a head, and other applicable components of a frame representing an individual. For example, the pose of the patient 104 depends on the relative angle and/or position of the arms and legs of the patient 104 to the trunk of the patient 104, as well as the angle of the arms and legs of the patient 104 to the direction of gravity within the clinical setting. In some implementations, the pose of the patient 104 is indicative of whether at least one foot of the patient 104 is angled toward the foot of the support structure 116. For example, the pose of the patient 104 may indicate "foot drop" of the patient 104. In particular implementations, the risk tracking system 126 estimates a frame associated with a recognized object in the images, wherein the object depicts an individual. The frame, in some cases, overlaps with at least a portion of the skeleton of the individual. The frame includes one or more beams and one or more joints. Each beam is represented by a line that extends from at least one joint. In some cases, each beam is represented as a line segment with a fixed length. In some implementations, each beam is presumed to be rigid (i.e., nonbending), but implementations are not so limited. In an example frame, beams may represent a spine, a trunk, a head, a neck, a clavicle, a scapula, a humerus, a radius and/or ulna, a femur, a tibia and/or fibula, a hip, a foot, or any combination thereof. Each joint may be represented as a point and/or a sphere. For example, an individual joint is modeled as a hinge, a ball and socket, or a pivot joint. In an example frame, joints may represent a knee, a hip, an ankle, an elbow, a shoulder, a vertebra, a wrist, or the like. In various implementations, the frame includes one or more keypoints. Keypoints include joints as well as other components of the frame that are not connected to any beams, such as eyes, ears, a nose, a chin, or other points of reference of the individual. The risk tracking system 126 can use any suitable pose estimation technique, such as PoseNet from TensorFlow. In various implementations, the risk tracking system 126 identifies the pose of the patient 104, the care provider 106, the visitor 108, or any other individual depicted in the images captured by the camera(s) 102.

The risk tracking system 126 may perform any of the image analysis techniques described herein using a computing model, such as a machine learning (ML) model. As used herein, the terms "machine learning," "ML," and their equivalents, may refer to a computing model that can be optimized to accurately recreate certain outputs based on certain inputs. In some examples, the ML models include deep learning models, such as convolutional neural networks (CNN). The term Neural Network (NN), and its equivalents, may refer to a model with multiple hidden layers, wherein the model receives an input (e.g., a vector) and transforms the input by performing operations via the hidden layers. An individual hidden layer may include multiple "neurons," each of which may be disconnected from other neurons in the layer. An individual neuron within a particular layer may be connected to multiple (e.g., all) of the neurons in the previous layer. A NN may further include at least one fully-connected layer that receives a feature map output by the hidden layers and transforms the feature map into the output of the NN

As used herein, the term "CNN," and its equivalents and variants, may refer to a type of NN model that performs at least one convolution (or cross correlation) operation on an input image and may generate an output image based on the convolved (or crosscorrelated) input image. A CNN may include multiple layers that transforms an input image (e.g., an image of the clinical setting) into an output image via a convolutional or crosscorrelative model defined according to one or more parameters. The parameters of a given layer may correspond to one or more filters, which may be digital image filters that can be represented as images (e.g., 2D images). A filter in a layer may correspond to a neuron in the layer. A layer in the CNN may convolve or cross correlate its corresponding filter(s) with the input image in order to generate the output image. In various examples, a neuron in a layer of the CNN may be connected to a subset of neurons in a previous layer of the CNN, such that the neuron may receive an input from the subset of neurons in the previous layer, and may output at least a portion of an output image by performing an operation (e.g., a dot product, convolution, cross-correlation, or the like) on the input from the subset of neurons in the previous layer. The subset of neurons in the previous layer may be defined according to a "receptive field" of the neuron, which may also correspond to the filter size of the neuron. U-Net (see, e.g., Ronneberger, et al., arXiv:1505.04597v1, 2015) is an example of a CNN model.

The risk tracking system 126 may include an ML model that is pre-trained based on training images that depict the features, as well as indications that the training images depict the features. For example, one or more expert graders may review the training images and indicate whether they identify the features in the training images. Data indicative of the training images, as well as the gradings by the expert grader(s), may be used to train the ML models. The ML models may be therefore trained to identify the features in the images obtained by the camera(s) 102. The features may be associated with identifying objects (e.g., recognizing the shape of the support structure 116 in the images), identifying movements (e.g., recognizing seizures or violent movements by individuals depicted in the images), identifying the pose of an individual depicted in the images, or identifying other visual characteristics associated with risks to individuals in the clinical setting.

In some cases, the risk tracking system 126 may rely on another type of data to detect, identify, and track objects in the images captured by the camera(s) 102. In various implementations, the other type of data is used to determine and/or confirm the position and/or movement of a subject being monitored by the risk tracking system 126.

According to some examples, the risk tracking system 126 detects, identifies, and tracks an object based on data received from the medical device 114. For example, the medical device 114 may generate data indicative of one or more parameters of the patient 104 and may transmit the data to the risk tracking system 126 over the communication network(s) 128. In various implementations, the risk tracking system 126 confirms the position, movement, or pose of a subject depicted in the images captured by the camera(s) 102 based on the data from the medial device 114. For example, the risk tracking system 126 may confirm that the patient 104 is located in the vicinity of the medical device 114 based on the medical device 114 capturing the parameter(s) of the patient 104. In some cases, the risk tracking system 126 can confirm that the patient 104 is moving by determining that the medical device 114 has detected an increasing heart rate of the patient 104.

In various implementations, the risk tracking system 126 may rely on data generated by the load cell(s) 118, the moisture sensor(s) 120, the temperature sensor(s) 122, the microphone(s) 124, or any combination thereof, to detect, identify, and track objects in the images captured by the camera(s) 102. For example, the data generated by the load cell(s) 118 may confirm that the patient 104 or some other subject is in contact with and/or supported by the support structure 116. In some particular cases, the load cell(s) 118 may confirm the presence of the patient 104 even when the patient 104 is obscured from view from the camera(s) 102, such as the patient 104 is hidden behind a sheet or is obscured by some other object in the scene monitored by the camera(s) 102. In some instances, the data generated by the moisture sensor(s) 120 confirms that the patient is disposed on the support structure 116 and/or a drink of the food item 110 has been spilled on the support structure 116. In some cases, the temperature sensor(s) 112 confirm that the patient 104, the care provider 106, the visitor 108, or some other heat-producing subject is in contact with the support structure 116. According to some implementations, the data generated by the microphone(s) 124 can be used to confirm the proximity of a subject producing noise (e.g., the medical device 114 producing an audible alarm and/or the patient 104, the care provider 106, or the visitor 108 talking) based on the volume of the sound detected by the microphone(s) 124.

In some examples, the environment 100 may also include an electronic medical record (EMR) system 130 that is configured to transmit data indicative of an EMR of the patient 104 to the risk tracking system 126 over the communication network(s) 128. As used herein, the terms "electronic medical record," "EMR," "electronic health record," and their equivalents, can refer to a data indicating previous or current medical conditions, diagnostic tests, or treatments of a patient. The EMRs may also be accessible via computing devices operated by care providers. In some cases, data stored in the EMR of a patient is accessible to a user via an application operating on a computing device. For instance, patient data may indicate demographics of a patient, parameters of a patient, vital signs of a patient, notes from one or more medical appointments attended by the patient, medications prescribed or administered to the patient, therapies (e.g., surgeries, outpatient procedures, etc.) administered to the patient, results of diagnostic tests performed on the patient, patient identifying information (e.g., a name, birthdate, etc.), or any combination thereof. In various implementations, the risk tracking system 126 uses the EMR data received from the EMR system 130 to detect, identify, and track subjects in the clinical setting. For example, the risk tracking system 126 may identify the patient 104 depicted in the images captured by the camera(s) 102 based on the EMR data indicating the identity of the patient 104. In some implementations, the risk tracking system 126 confirms that the patient 104 is fully supported by the support structure 116 by determining that the weight of the patient 104 indicated in the EMR data is consistent with the weight detected by the load cell(s) 118 of the support structure 116.

According to some implementations, the environment 100 may further include a location tracking system 132. In some cases, the location tracking system 132 is at least a component of a real time location system (RTLS) configured to detect the location of various tags in the clinical setting. The location tracking system 132, for example, includes multiple fixed-position sensors configured to communicate with various tags disposed in the clinical setting. In some cases, the sensors receive a wireless signal (e.g., an NFC signal, radio frequency (RF) signal, etc.) broadcast from a tag. The location tracking system 132 determines the times at which the wireless signal was received by the sensors from the tag. Based on a discrepancy of the times at which the signal was received, and the positions of the sensors, the position of the tag can be derived (e.g., using triangulation). According to various implementations, the location of the tag is tracked over time as the tag broadcasts additional signals received by the sensors.

In various examples, the location tracking system 132 is configured to identify the locations of a tag associated with the patient 104, a tag associated with the care provider 106, a tag associated with some other type of object, or any combination thereof. For instance, the tag associated with the patient 104 may be integrated into an identification bracelet worn by the patient 104. In some examples, the tag associated with the care provider 106 may be integrated into a badge 134, clothing, equipment, or other wearable item that is worn and/or carried by the care provider 106. The badge 134, for example, may be an identification badge of the care provider 106. In some cases, the location tracking system 132 detects the location of the food item 110, the movable object 112, the medical device 114, the support structure 116, or some other object in the environment 100, based on one or more tags integrated with and/or attached to the food item 110, the movable subject 112, the medical device 114, the support structure 116, or the other object. In various cases, the location tracking system 132 may generate data indicating the location of at least one tag associated with at least one of the patient 104, the care provider 106, or another subject in the clinical setting. The location tracking system 132 may transmit the data to the risk tracking system 126. Accordingly, the risk tracking system 126 may confirm the position, movement, or identity of a subject based on the location of a tag associated with the subject as indicated by the location tracking system 132.

The risk tracking system 126, in various cases, may also detect, identify, and track objects based on data received from at least one wearable sensor 136. The wearable sensor(s) 136, for example, may be worn by one or more individuals, such as the patient 104 and/or care provider 106. In some examples, the wearable sensor(s) 136 include one or more accelerometers configured to detect an acceleration of the wearable sensor(s) 136. In some cases, the wearable sensor(s) 136 include one or more gyroscopes configured to detect an orientation and/or angular velocity of the wearable sensor(s) 136. In some cases, the wearable sensor(s) 136 detect a vital sign or other physiological parameter of the patient 104 and/or care provider 106. For example, the wearable sensor(s) 136 may include a temperature sensor configured to detect a temperature of the patient 104 and/or care provider 106, a heart rate sensor configured to detect a heart rate of the patient 104 and/or care provider 106, an oximetry sensor configured to detect a blood oxygenation of the patient 104 and/or care provider 106, or any combination thereof. In various implementations, the wearable sensor(s) 136 may include and/or be integrated with a watch, a bracelet, a necklace, clothing, equipment, patch, or other wearable device that is worn by or otherwise affixed to the patient 104 and/or the care provider 106. The wearable sensor(s) 136 may generate and transmit data indicative of one or more detected parameters to the risk tracking system 126 over the communication network(s) 128. In some cases, the risk tracking system 126 utilizes the data from the wearable sensor(s) 136 to confirm the position, movement, or pose of an object in the images captured by the camera(s) 102. For example, the risk tracking system 126 may confirm that the care provider 106 is moving based on an acceleration detected by the wearable sensor(s) 136.

In some cases, the risk tracking system 126 detects, identifies, or tracks a subject based on data from another type of sensor. For instance, the moveable subject 112 includes a sensor 138. In some cases, the sensor 128 includes an accelerometer configured to detect an acceleration of the movable object 112. In some examples, the sensor 128 includes a gyroscope configured to detect an orientation and/or angular velocity of the movable object 112. In various cases, the movable object 112 includes wheels configured to facilitate transport of the movable object 112 throughout the clinical setting. The sensor 128 may include a distance sensor configured to detect a distance that the movable object 112 has moved along a path through the clinical setting and/or a rotation of the wheels as the movable object 112 is transported throughout the clinical setting. In some cases, the sensor 128 may include a speed sensor configured to detect the speed that the movable object 112 is traveling along the path through the clinical setting. The sensor 138 may be configured to generate data based on one or more parameters (e.g., acceleration, orientation, angular velocity, distance, speed, etc.) related to the movable object 112 and to transmit the data to the risk tracking system 126 over the communication network(s) 128. In some cases, the risk tracking system 126 may calculate the risk to the patient 104 and/or the care provider 106 based on the data from the sensor 138. Thus, the risk tracking system 126 may confirm a movement of the moveable object 112 based on data from the sensor 128.

In various implementations, the risk tracking system 126 identifies a risk to the patient 104 based on the position, movement, and pose of the patient 104 and/or other subjects depicted in the images captured by the camera(s) 102. For example, the risk tracking system 126 identifies one or more suspected events based on the images captured by the camera(s) 102 and/or data detected by other sensors in the environment 100. In some examples, the risk tracking system 126 detects and/or confirms the event(s) by determining a confidence level indicating the confidence that the event occurred, determining a location at which the event occurred, determining an acceleration and/or force associated with the event, and determining a time interval at which the suspected event occurs. Based on the event(s), the risk tracking system 126 may quantify the risk that the patient 104 has and/or will experience a particular type of injury and/or medically relevant concern. The risk, for example, is represented as a probability (e.g., a percentage) that corresponds to the likelihood that a particular experience has and/or will occur.

According to some cases, the risk tracking system 126 identifies a risk that the patient 104 has and/or will develop a pressure injury. As used herein, the term "pressure injury," and its equivalents, may refer to localized damage to the skin and/or underlying tissue of a patient, and can be caused by rubbing and/or pressure exerted on the skin over time. In various implementations, the risk tracking system 126 identifies the pressure injury risk based on analyzing the images captured by the camera(s) 102. The risk tracking system 126 may determine the risk that the patient 104 will develop any pressure injury and/or determine the risk that the patient 104 will develop a pressure injury on a particular area of the patient's 104 body (e.g., the back, a knee, etc.).

In some cases, the risk tracking system 126 determines the pressure injury risk by identifying and/or tracking a subject on which the patient 104 disposed against and/or is resting their weight (e.g., between the patient 104 and the support structure 116). In various cases, the risk tracking system 126 identifies a fold in a sheet extending from underneath the patient 104 and can therefore infer that the fold is disposed between the patient 104 and the support structure 116. The fold may exert an area of increased pressure on the patient 104 (e.g., compared to an unfolded sheet) and can therefore increase the patient's 104 risk of developing a pressure injury. The risk tracking system 126 may determine the pressure injury risk based on the presence of other subject disposed on the skin of the patient 104. In some examples, the risk tracking system 126 identifies a subject disposed on the skin of the patient 104 (e.g., a feeding tube, a blood pressure cuff, a sequential compression device (SCD), another type of medical device, etc.) based on the images captured by the camera(s) 102. The risk of the pressure injury may increase based on the presence and/or time that the subject is disposed on the skin of the patient 104.

According to various implementations, the risk tracking system 126 determines the pressure injury risk based on a body shape and size of the patient 104. For example, the risk tracking system 126 may estimate a volume of the patient 104 based on the images captured by the camera(s) 102. The pressure injury risk may be heightened if the patient 104 has a greater than threshold volume and/or a lower than a threshold volume. The risk tracking system 126 may determine that the patient 104 has a bony protrusion and may determine the pressure injury risk based on the presence of the bony protrusion and/or the time that the protrusion is in contact with another subject in the clinical setting. As used herein, the term "bony protrusion," and its equivalents, refers to a portion of an individual's body wherein a bone is disposed under skin with minimal to no soft tissue therebetween. Examples of bony protrusions include elbows, ankles, and the like. The risk tracking system 126 may identify bony protrusions using image recognition techniques on images depicting the bony protrusion. According to some examples, the risk tracking system 126 determines a body habitus of the patient 104 and determines the pressure injury risk based on the body habitus. For example, the patient 104 may have a heightened risk of a sacrum pressure injury if the patient 104 carries extra weight on their hips. The risk tracking system 126 may determine that the patient 104 is carrying extra weight on their hips by determining that the width of the patient's 104 hips is greater than a threshold width.

The risk tracking system 126 may determine the pressure injury risk based on the pose of the patient 104. Some poses are associated with an increased likelihood of developing a pressure injury, such as a supine position, a lateral position, a prone position, legs crossed, heels crossed, a side position, a position in which the patient 104 is performing greater than a threshold amount of plantar flexion (e.g., an angle between the foot of the support structure 116 and at least one foot of the patient 104 is less than a threshold angle), or a position in which one knee is resting on another knee. Poses associated with a lowered risk of developing an injury include poses in which the patient's 104 heels are elevated (e.g., using a pillow, sling, or boots), the patient 104 has a pillow or other support disposed between their legs, and so on.

The risk tracking system 126, in some examples, determines the pressure injury risk based on a movement of the patient 104. As used herein, the term "movement" and its equivalents may refer to a displacement, a velocity, a speed, an acceleration, a jerk, or any other differential of position. In some implementations, the risk tracking system 126 detects a turn of the patient 104 (e.g., while resting in the support structure) and determines the pressure injury risk based on the turn. For instance, the risk tracking system 126 determines the pressure injury risk based on a frequency of turns made by the patient 104 over a time interval (wherein a low frequency of turns is associated with a heightened pressure injury risk). The risk tracking system 126 may quantify the movement of the patient 104 over a time interval and may determine the pressure injury risk based on the amount of the movement.

In some cases, the risk tracking system 126 determines the pressure injury risk based on the time spent in the support structure 116 using the images detected by the camera(s) 102. For example, the risk tracking system 126 determines the pressure injury risk based on the amount of time that the patient 104 is in contact with another subject and/or in a particular pose.

The risk tracking system 126, in some cases, determines the pressure injury risk based on detecting a shear event using the images detected by the camera(s) 102. The term "shear event," and its equivalents, can refer to an event in which a shear force is generated as multiple subjects rub against each other. In some examples, the risk tracking system 126 identifies the shear event by identifying movement of the patient 104 with respect to another subject (e.g., the support structure 116) and also identifying that the patient 104 is in contact with the other subject during the movement. For instance, the care provider 106 may pull the patient 104 up in the support structure 116, which may exert a shear force on the skin of the patient 104 and increase the risk of the pressure injury. In some cases, the risk tracking system 126 assesses the severity of the shear event based on inferring a force between the patient 104 and the other subject. For example, the risk tracking system 126 may infer that the weight of the patient 104 is pressing down on the support structure 116 when the risk tracking system 126 determines that the patient 104 is resting on the support structure 116.

In some examples, the risk tracking system 126 determines a vital sign of the patient 104 based on the images captured by the camera(s) 102 and determines the pressure injury risk based on the vital signs. For example, the camera(s) 102 are configured to obtain infrared images of the patient 104 and the risk tracking system 126 utilizes image processing techniques to identify the heart rate, respiration rate, and blood oxygenation (e.g., SpO₂) of the patient 104 based on the infrared images. The patient 104 may have a heightened risk of developing a pressure injury if the heart rate and/or respiration rate are relatively low (e.g., below a first threshold) and/or relatively high (e.g., above a second threshold). The patient 104 may have a heightened risk of developing a pressure injury if the SpO₂ is relatively low (e.g., below a third threshold).

According to some instances, the risk tracking system 126 determines the pressure injury risk based on recognizing toileting and/or incontinence assistance from the care provider 106. The risk may increase based on moisture disposed between the patient 104 and another subject (e.g., the support structure 116). Accordingly, the risk tracking system 126 may determine the risk based on a frequency at which the care provider 106 provides toileting assistance to the patient 104 and/or a frequency at which the care provider 106 is changing incontinence pads or briefs of the patient 104. The risk tracking system 126, in some cases, identifies toileting assistance and/or changing events based on analyzing the images captured by the camera(s) 102.

The risk tracking system 126 may further rely on other sources of data to identify the pressure injury risk. In various cases, the risk tracking system 126 determines the pressure injury risk based on at least one of the following factors noted in the EMR data received from the EMR system 130: a medication of the patient 104 (e.g., a vasopressor), whether the patient 104 is using briefs or other incontinence management devices, diagnoses of the patient 104 (e.g., diabetes, diarrhea, etc.), demographics of the patient 104 (e.g., age, skin tone, BMI, etc.), measures of mobility over time, medical devices used in the care of the patient 104 (e.g, a cast, a splint, a cervical collar, a continuous positive airway pressure (CPAP) and/or a bilevel positive airway pressure (BiPap) mask, an endotracheal tube, a feeding tube, a Foley-catheter, a cervical spine brace (e.g., a halo), a nasal oxygen device (e.g., including a tube disposed in an ear and/or nose of the patient 104), an SCD, a tracheostomy neck plate, etc.), a nutritional intake of the patient 014, or a history of pressure injuries experienced by the patient 104. In some cases, the EMR data includes a note from a care provider (e.g., the care provider 106) indicating the history of pressure injuries. In some cases, the note is sent from a separate device in the environment 100.

According to some implementations, the risk tracking system 126 utilizes one or more parameters (e.g., heart rate, respiratory rate, SpO₂, etc.) detected by the medical device 114 to determine the pressure injury risk. In some cases, the risk tracking system 126 determines the pressure injury risk based on data from the support structure 116. For example, pressures detected from the load cell(s) 118, moisture detected by the moisture sensor(s) 120, a temperature detected by the temperature sensor(s) 112, or any combination thereof, can be used to determine the pressure injury risk. In addition, the data from the medical device 114 and/or the support structure 116 may be used by the risk tracking system 126 to determine the time that a pressure was exerted on the patient 104, moisture was present on the patient 104, or between movements of the patient 104 (e.g., on the support structure 116).

In various instances, the risk tracking system 126 identifies a risk that the patient 104 has participated and/or will participate in an unauthorized bed exit. The risk tracking system 126 may identify one or more events based on object tracking of the images captured by the camera(s) 102. The event(s) include, for instance, railings on the support structure 116 being lowered, sheets on the patient 104 being moved and/or removed, legs of the patient 104 rotating, the patient 104 sitting up, the legs of the patient 104 being hooked around an edge of the support structure 116, the patient 106 moving toward an edge of the support structure 116, the patient 106 standing up, or a combination thereof. Subjects corresponding to objects that can be tracked to identify the event(s) include, for example, the patient 104, the legs of the patient 104, a sheet on the support structure 116, the feet of the patient 104, the hands of the patient 104, the head of the patient 104, or any combination thereof. In some cases, the risk tracking system 126 identifies the risk that the patient 104 has participated in an unauthorized bed exit based on the pose of the patient 104 over time.

In some examples, the risk tracking system 126 further identifies the risk that the patient 104 has participated and/or will participate in an unauthorized bed exit based on other sources of data. The risk tracking system 126 may determine that the patient 104 is not authorized to exit the support structure 116 based on EMR data received from the EMR system 130. For example, the patient 104 may have a heightened falls risk or some other malady that prevents them from safely exiting the support structure 116. The risk tracking system 126 may confirm that the patient 104 has exited the support structure 118 by receiving data from the medical device 114 indicating that a parameter of the patient 104 is no longer detected (e.g., that the patient 104 has removed a wired sensor of the medical device 114 in order to accommodate the patient 104 leaving the support structure 116). In some cases, the risk tracking system 126 confirms that the patient 106 is moving on the support structure 116 based on a shift in weight detected by the load cell(s) 118 of the support structure 116. The risk tracking system 126 may confirm that the patient 106 has left the support structure 116 based on a decrease in weight detected by the load cell(s) 118 of the support structure 116.

In some cases, the risk tracking system 126 identifies a risk that the patient 104 has and/or will aspirate. In various implementations, the risk tracking system 126 identifies the risk based on a pose of the patient 104. The risk of aspiration increases, for instance, if the angle of the neck of the patient 104 is within a particular range and/or the head of the patient 104 is lower than a particular elevation with respect to the rest of the patient's 104 body. In some examples, the support structure 116 is configured to elevate the head of the patient 104 but the risk of aspiration increases if the patient's 104 body slides down the support structure 116 over time. For example, the risk tracking system 126 determines the risk that the patient 104 will aspirate based on the angle of the patient's 104 neck, the elevation of the patient's 104 head with respect to the elevation of the patient's 104 trunk or torso, and whether the patient 104 has slid down the support structure 116.

The risk tracking system 126 may use other types of data to determine and/or confirm the risk that the patient 104 will aspirate. For instance, the risk tracking system 126 determines the risk is heightened based on the EMR data from the EMR system 130 indicating that the patient 104 has pneumonia or other respiratory problems, the patient 104 is sedated or unconscious, or the patient 104 has a physical injury that prevents the patient 104 from moving freely. In some examples, a change in blood pressure detected by the medical device 114 indicates that the orientation of the patient 104 has changed, which the risk tracking system 126 can use to confirm a change in the pose of the patient 104. In some cases, the risk tracking system 126 determines the risk based on parameters detected by the support structure 116. For example, an increase in weight detected by a load cell 118 at the head of the support structure 116 and an increase in weight detected by a load cell 118 at the foot of the support structure 116 may be indicative of the patient 104 sliding down in the support structure 116. In some cases, the support structure 116 itself can detect whether it is configured to elevate the head of the patient 104.

In some examples, the risk tracking system 126 identifies a risk that the patient 104 has and/or will experience a fall. In various implementations, the risk increases based on an elevation of the patient 104 with respect to the support structure 116 and/or the floor. For example, the patient 104 has a relatively low falls risk if the patient 104 is resting on the support structure 116 or sitting on the floor, but a relatively higher falls risk if the patient 104 is standing up, unsupported. In some cases, the risk tracking system 126 determines the falls risk based on a movement of the patient 104. For example, the risk tracking system 126 may determine that the patient 104 has a heightened falls risk if the patient 104 is moving stochastically (e.g., shaking) or in an unbalanced fashion while the pose of the patient 104 is upright. In some cases, the risk tracking system 126 determines that the patient 104 is at a heightened risk of a fall by determining that the patient 104 has exited the support structure 116.

According to various implementations, the risk tracking system 126 may further identify the falls risk based on other data sources. For example, the risk tracking system 126 may determine that the patient 104 has a heightened falls risk based on EMR data from the EMR system 130 indicating that the patient 104 has had a history of falls, seizures, vasovagal episodes, is taking a medication that increases the likelihood of a fall, or is of an age over a threshold age. In some cases, the EMR data indicates that the patient 104 is a prone to injuries from falls, such as the patient 104 has osteoporosis or is taking a blood thinner medication. The risk tracking system 126 may determine that the patient 104 is at a heightened risk for a fall if the medical device 114 detects a blood pressure under a threshold blood pressure and/or a heart rate under a threshold heart rate.

In some cases, the risk tracking system 126 identifies a risk that the patient 104 has experienced a seizure. The risk tracking system 126 determines that the risk is heightened, for example, by determining that the pose of the patient 104 is stiff and/or indicates that the patient 104 has fallen to the floor. In some cases, the risk tracking system 126 determines that the risk is heightened by determining that a face of the patient 104 is consistent with a facial expression associated with seizure (e.g., the patient's 104 eyes are rolled and/or mouth is making chewing motions). In various cases, the risk tracking system 126 determines that the risk is heightened by determining that the motion of the patient 014 is consistent with twitching.

The risk tracking system 126 may rely on other types of data to determine the risk that the patient 104 has experienced a seizure. The risk tracking system 126 may determine that the patient 104 is at a heightened risk for developing a seizure based on the EMR data from the EMR system 130 indicating that the patient 104 has had a previous diagnosis of epilepsy, brain injury, or sepsis. In various examples, the risk tracking system 126 determines the risk based on an EEG, heart rate, respiration rate, temperature, or blood pressure detected by the medical device 114 during the suspected seizure event. In some cases, the risk tracking system 126 determines that the patient 104 has a heightened risk if the moisture sensor(s) 120 detect moisture on the support structure 116, which may indicate that the patient 104 has urinated.

The risk tracking system 126, in some examples, identifies a risk that the patient 104 has been visited by an unauthorized visitor. In various implementations, the risk tracking system 126 determines the risk based on detecting, identifying, and tracking the visitor 108 relative to the patient 104. For example, the risk tracking system 126 may conclude that the risk is relatively high if the patient 104 is not permitted to have a visitor and the visitor 108 enters the room of the patient 104. In some implementations, the risk tracking system 126 determines that the visitor 108 is unauthorized by determining that the EMR data from the EMR system 130 indicates that the patient 104 is not permitted visitors.

In some implementations, the risk tracking system 126 identifies a risk that the patient 104 is participating and/or will participate in unauthorized eating or drinking. The risk tracking system 126 may determine the risk by tracking the patient 104 and the food item 110. For example, the risk tracking system 126 determines the risk is heightened by determining that at least a portion of the food item 110 is in the room of the patient 104, is being held by the patient 104, or has entered the mouth of the patient 104. In some cases, the risk tracking system 126 confirms that the patient 104 is not authorized to eat or drink based on the EMR data from the EMR system 130 indicating that the patient 104 is "nothing-by-mouth" or "NPO."

In various cases, the risk tracking system 126 identifies a risk that the patient 104 has absconded and/or will abscond. For instance, the risk tracking system 126 may determine that the risk is heightened by determining that the patient 104 has approached and/or is passing through a doorway or other threshold to the room of the patient 104. In some cases, the risk tracking system 126 confirms that the patient 104 is at a heightened risk of absconding by determining, based on the EMR data, that the patient 104 has dementia, the patient 104 is a suspect in a criminal investigation, and/or is otherwise a flight risk.

According to some cases, the risk tracking system 126 identifies a risk that the patient 104 has experienced and/or will experience a moment of wakefulness. This determination may be particularly relevant for sedated and/or unconscious patients, such as patients in intensive care environments. The risk tracking system 126, for example, determines the risk based on a detected movement of the patient 104 (e.g., as the patient 104 is resting on the support structure 116). In some cases, the risk tracking system 126 determines that the patient 104 is at a heightened risk of wakefulness by determining that the pose of the patient 104 indicates the patient is sitting or standing upright. In various cases, the risk tracking system 126 determines that the patient is at a heightened risk by determining, based on the EMR data from the EMR system 130, that a sedative was administered to the patient 104 greater to a threshold time ago and is therefore likely to be wearing off. In some cases, the risk tracking system 126 predicts the wakefulness based on a heightened heart rate or respiratory rate detected by the medical device 114. Movements consistent with wakefulness can be further confirmed by shifts in weight detected by the load cell(s) 118 and/or sounds (e.g., groaning or voice sounds) detected by the microphone(s) 124 of the support structure 116.

In some implementations, the risk tracking system 126 identifies a risk that the patient 104 has self-harmed and/or will self-harm. For instance, the risk tracking system 126 may determine that the movement of the patient 104 is consistent with self-harm and/or that the EMR data from the EMR system 130 indicates that the patient 104 is at risk for self-harm (e.g., the patient 104 is on a psychiatric hold, the patient 104 has a history of self-harm, etc.).

According to various implementations, the risk tracking system 126 identifies a risk to the care provider 106 based on the position, movement, and pose of the care provider 106 and/or other subjects depicted in the images captured by the camera(s) 102.

In various cases, the risk tracking system 126 identifies a risk that the care provider 106 has and/or will experience an act of violence against the care provider 106. In various examples, the risk tracking system 126 identifies a violent act by the patient 104 and/or the visitor 108 against the care provider 106 by monitoring the position, movement, and pose of the patient, visitor 108, and the care provider 106. For instance, the risk tracking system 126 may determine that there is a heightened risk that the care provider 106 has experienced a violent act when a limb of the patient 104 and/or the visitor 106 exceeds a threshold velocity, comes into contact with the care provider 106, and/or the care provider 108 moves in a way consistent with a transfer of momentum from the limb of the patient 104 and/or the visitor 108 to the care provider 106. In some examples, the risk tracking system 126 confirms and/or determines the risk of the violence based on sounds (e.g., indicative of yelling, pain, or distress) detected by the microphone(s) 124.

In some implementations, the risk tracking system 126 identifies a risk that the care provider 106 will develop a repetitive use injury. In various cases, the risk tracking system 126 determines the risk by counting the number of times that the care provider 106 performs a particular motion (e.g., during a time interval, such as a workday of the care provider 106) in the images obtained by the camera(s) 102. The risk may increase as the number of times that the care provider 106 performs the particular motion increases. In some cases, the type of motion that is repeated can further impact the risk of injury. Repetitive motions with a high likelihood of causing injury include extreme motions, wherein the limbs of the care provider 106 move greater than a threshold angle and/or distance from a center line that is defined along the spine of the care provider 104. For example, repetitive motions that involve the care provider 106 moving an arm above shoulder-level may be particularly likely to cause injury. According to some implementations, the motion of the care provider 106 is further determined and/or confirmed based on an acceleration or other parameter detected by the wearable sensor(s) 136.

In various examples, the risk tracking system 126 identifies a risk that the care provider 106 will develop a posture-based injury. For example, the risk tracking system 126 may identify the risk by detecting that the pose of the care provider 106 is in a predetermined risky pose for greater than a threshold period of time. Examples of risky poses include slouching (e.g., the spine of the care provider 106 has greater than a threshold curvature), sitting, and the like. The risk of the posture-based injury, for example, may increase as the amount of time that the care provider 106 adopts the risky pose (e.g., continuously and/or non-continuously). The risk tracking system 126 may determine and/or confirm the risky pose of the care provider 106 based on various parameters detected by the wearable sensor(s) 136 (e.g., acceleration).

According to some cases, the risk tracking system 126 identifies a risk that the care provider 106 will develop a lifting injury. In various implementations, the risk tracking system 126 assesses this risk by analyzing the images captured by the camera(s) 102. In some cases, the risk tracking system 126 determines the risk based on the pose of the care provider 106 during a lifting event. For example, the care provider 106 may be at a heightened risk for developing a lifting injury if the pose of the care provider 106 indicates that the care provider 106 is lifting a subject (e.g., the moveable subject 112 or the patient 104) using a limb that is greater than a threshold angle and/or distance from the trunk of the care provider 106, that the spine of the care provider 106 is curved during the lifting event, that the care provider 106 is "lifting with their back" (e.g., placing a significant load on weak muscles in the back of the care provider 106 rather than strong muscles associated with the posterior chain), or the like. The risk tracking system 126 may determine the risk based on the distance at which the subject is being lifted. For example, the risk tracking system 126 may determine the vertical distance that the care provider 106 lifts the moveable subject 112 by tracking an object corresponding to the moveable subject 112 in the images obtained by the camera(s) 102 and determine the risk accordingly.

In some examples, the risk tracking system 126 determines the weight of the subject being lifted by the care provider 106 and determines the risk based on the weight. The risk tracking system 126 may determine the weight, contextually, based on analyzing the images captured by the camera(s) 102. For example, the risk tracking system 126 may infer the weight of the moveable subject 112 by determining that the care provider 106 is stable (e.g., not falling over), the pose of the care provider 106 carrying the moveable subject 112 is out-of-alignment, the weight of the care provider 106, and a free body diagram estimation of the gravitational force on the moveable subject 112 that would enable the out-of-alignment care provider 106 to be stable. According to some cases, the risk tracking system 126 infers the weight by detecting a facial expression associated with straining on the face of the care provider 106. In some cases, the risk tracking system 126 stores the weights of various subjects in the clinical setting in a local database. For example, the risk tracking system 126 may identify the care provider 106 based on the images and look up the weight of the care provider 106 stored in database. In some cases, the risk tracking system 126 may identify the moveable subject 112 in the images as an x-ray machine (e.g., based on a fiducial marker attached to and identifying the moveable subject 112 and/or the shape of the moveable subject 112) and may look up the weight of the x-ray machine in the database.

According to some examples, the risk tracking system 126 may determine the risk of the lifting injury based on whether the care provider 106 uses an assistive lifting device and/or is assisted by the help of another individual in performing the lift. For example, the EMR data from the EMR system 130 may indicate that the patient 104 should be lifted and/or moved with at least two people (e.g., a "two-person assist"). If the risk tracking system 126 determines that the care provider 106 moves the patient 104 without the assistance of another individual or an assistive device, then the risk tracking system 126 may determine that the care provider 106 is at a heightened risk of a lifting injury.

In various implementations, the risk tracking system 126 may rely on other sources of data to determine the care provider's 106 risk of the lifting injury. In some cases, one or more parameters detected by the wearable sensor(s) 136 can be used to derive the movement and/or strain of the care provider 106 during the lifting event. In some cases, the sensor 138 on the moveable subject 112 detects a metric indicative of the movement of the moveable subject 112 during the lifting event.

According to some implementations, the risk tracking system 126 adjusts the risk of the repetitive use injury, the posture-based injury, or the lifting injury based on health information associated with the care provider 106. For example, the risk tracking system 126 may include a database that stores a height, a weight, a body mass index (BMI), a health condition (e.g., asthma, pregnancy, etc.) of the care provider 106, a history of previous injuries, and so on. The risk tracking system 126 may adjust the risk to the care provider 106 based on the health information.

The risk tracking system 126 may generate a report and/or alert based on the risk to the patient 104 and/or the care provider 106. For example, the risk tracking system 126 generates the report and/or alert to indicate a risk of injury to the patient 104 and/or the care provider 106. In particular instances, the risk tracking system 126 generates the report and/or alert based on determining that a risk to the patient 104 and/or a risk to the care provider 106 exceeds a threshold. In some implementations, the risk tracking system 126 may output the report and/or alert to various devices, such as a clinical device 140, an administrator device 142, a security device 144, or a monitoring terminal 146. The clinical device 140, the administrator device 142, the security device 144, or the monitoring terminal 146 may output the report and/or alert by visually displaying the report and/or report on a display screen, audibly outputting the report and/or alert via a speaker, haptically outputting the report and/or alert via vibration, or any combination thereof.

The clinical device 140 may be a computing device associated with the care provider 106 or some other care provider within the environment 100. A computing device may include at least one processor configured to perform operations. In some cases, the operations are stored in memory in an executable format. Examples of computing devices include a personal computer, a tablet computer, a smart television (TV), a mobile device, a mobile phone, or an Internet of Things (IoT) device.

The administrator device 142 may be a computing device that is accessible by a manager or administrator within the clinical setting. In various cases, the manager or administrator can use the report or alert to make track dangerous conditions and injuries within the clinical setting. In some cases, the manager or administrator can use the report or alert to make management decisions about the clinical setting, such as increasing or decreasing care provider staffing, hiring decisions, ordering decisions (e.g., ordering more medical devices or assistive equipment within the clinical setting), and the like. In some cases, the risk tracking system 126 generates and the administrator device 142 receives an alert or report that is indicative of the risk of multiple patients (including the patient 104) or multiple care providers (including the care provider 106), which may provide context about overall conditions of the clinical setting.

The security device 144 may be associated with a security team and/or security guard of the clinical setting. In some cases, the risk to the patient 104 or the care provider 106 can be addressed by the security team and/or security guard instead of, or in addition to, assistance from the care provider 106. For example, the alert may be transmitted to the security device 144 upon the risk tracking system 126 determining that there is greater than a threshold risk of the patient 104 absconding, an unauthorized visitor of the patient 104 is present, or that a violent act has been carried out against the care provider 106. Accordingly, the security team and/or security guard may receive the report or alert and make take appropriate action based on the report or alert.

The monitoring terminal 146, in various cases, may be associated with a monitor who is responsible for remotely monitoring the patient 104. In some cases, the monitor may be responsible for remotely monitoring multiple patients (including the patient 104), simultaneously. According to various implementations, the monitoring terminal 146 may simultaneously display multiple image and/or video feeds depicting the multiple patients under surveillance. The alert or report indicating the risk to the patient 104 may cause the monitoring terminal 146 to emphasize the image and/or video feed associated with the patient 104. Accordingly, the monitor's attention may be drawn to a potential adverse event experienced by the patient 104.

In some implementations, the risk tracking system 126 transmits the alert to the medical device 114. For instance, the medical device 114 may be an assistive device, such as a device configured to assist the care provider 106 with lifting the patient 104 or equipment. For example, the risk tracking system 126 may transmit the alert based on the risk that the care provider 106 has or will develop a lifting injury. The medical device 114 may output a notification (e.g., a reminder notification) to the care provider 106 based on the alert. The notification, for example, is a visual notification (e.g., a blinking light), an audible notification (e.g., a chime), or a combination thereof. Accordingly, the medical device 114 may remind the care provider 106 to use the assistive device prior to attempting a lift.

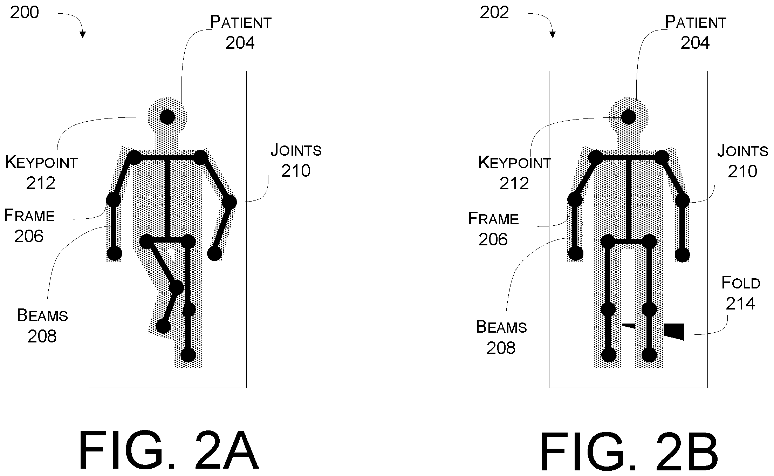

FIGS. 2A and 2B illustrate example images 200 and 202 of a patient 204 at risk for developing an injury. In some implementations, the images 204 are captured by one or more cameras, such as the camera(s) 102 described above with reference to FIG. 1. The patient 204, for example, is the patient 104 described above with reference to FIG. 1. The images 200 and 202 may be captured from a camera directly over the patient 204 as the patient 204 is resting on a support structure (e.g., the support structure 116 described above with respect to FIG. 1)

FIG. 2A illustrates a first image 200 of the patient 204 at a first time. As shown, an object depicting the patient 204 is overlaid with a frame 206 of the patient 204. The frame 206 includes multiple beams 208 and multiple joints 210. The frame 206 is overlaid over at least a portion of the skeleton of the patient 204. The beams 208 may represent bones in the skeleton. The joints 210 represent flexible connection points between the bones of the skeleton, such as musculoskeletal joints or sockets. In addition, the frame 206 includes a keypoint 212 representing the head and/or face of the patient 204. The frame 206 may be generated based on an image of the patient using a technique such as OpenPose.

In various implementations, a pressure injury risk and/or falls risk of the patient 204 may be determined based on the first image 200 and/or the frame 206. For example, the frame 206 in the first image 200 indicates that the joints 210 corresponding to the knees of the patient 204 are less than a threshold distance apart, which may indicate that the knees are touching. The patient 204 may therefore have a heightened risk for developing a pressure injury due to pressure and/or rubbing between the knees. In some cases, the position of the knees is indicative that the patient 204 is in the process of trying to exit the support structure. Accordingly, the pose of the patient 204 may also indicate that falls risk of the patient 204 is heightened, particularly if the patient 204 is not allowed to exit the support structure without assistance.

FIG. 2B illustrates a second image 202 of the patient 204 at a second time. As shown, the patient 204 has shifted position on the support structure such that the frame 206 is in a different configuration at the second time than at the first time. In particular, the frame 206 is associated with a relatively neutral pose, without the knees of the patient 204 touching each other. Therefore, in this respect, the contribution of the pose of the patient 204 to the pressure injury risk of the patient 204 may be lower at the second time than at the first time.

However, the second image 202 also depicts a fold 214 between the patient 204 and the support structure. For instance, the fold 214 may be in a sheet disposed between the patient 204 and the support structure. The presence of the fold 214 disposed between the patient 204 and the support structure may increase the pressure injury risk for the patient 204, particularly at a portion of the patient's 204 body disposed against the fold 214.

In some cases, the first image 200 and the second image 202 may indicate a movement of the patient 204 between the first time and the second time. The pressure injury risk of the patient 204 may further be based on the length between the first time and the second time. For example, frequent movements by the patient 204 may indicate a reduced risk of developing a pressure injury, whereas infrequent movements by the patient 204 may indicate a heightened risk of developing a pressure injury.

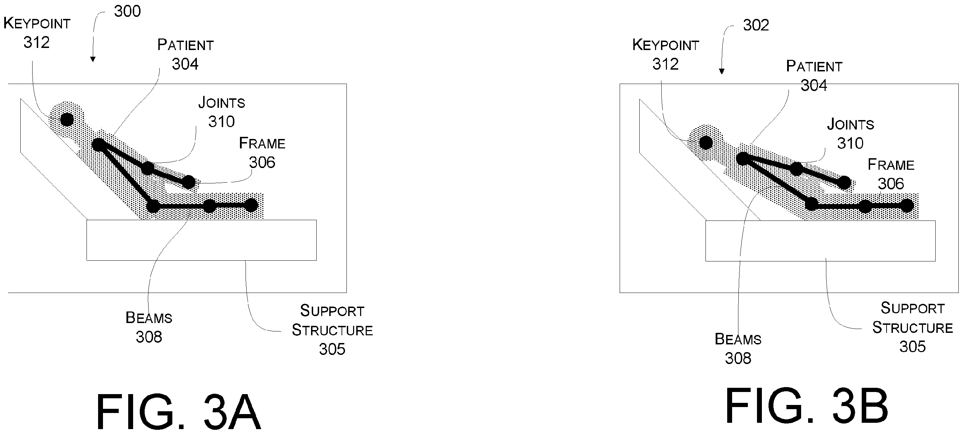

FIGS. 3A and 3B illustrate additional example images 300 and 302 of a patient 304 at risk for developing an injury. In some implementations, the images 300 and 302 are captured by one or more cameras, such as the camera(s) 102 described above with reference to FIG. 1. The patient 304, for example, is the patient 104 described above with reference to FIG. 1. The images 300 and 302 may be captured from a camera positioned to the side of the patient 304 as the patient 304 is resting on a support structure 305 (e.g., the support structure 116 described above with respect to FIG. 1)

FIG. 3A illustrates a first image 300 of the patient 304 at a first time. As shown, an object depicting the patient 304 is overlaid with a frame 306 of the patient 304. The frame 306 includes multiple beams 308 and multiple joints 310. The frame 306 is overlaid over at least a portion of the skeleton of the patient 304. The beams 308 may represent bones in the skeleton. The joints 310 represent flexible connection points between the bones of the skeleton, such as musculoskeletal joints or sockets. In addition, the frame 306 includes a keypoint 312 representing the head and/or face of the patient 304. The frame 306 may be generated based on an image of the patient using a technique such as OpenPose.

Based on the position of the patient 304 with respect to the support structure 305 and/or the configuration of the frame 306, the patient 304 is well-supported by the support structure 305 and the head of the patient 305 is elevated. Accordingly, the patient 304 may have a relatively low pressure injury risk and a relatively low aspiration risk.

FIG. 3B illustrates a second image 302 of the patient 304 at a second time. At the second time, based on the position of the patient 304 with respect to the support structure 305 and/or the configuration of the frame 306, both the pressure injury risk and the aspiration risk of the patient 304 may be increased. For example, the angle between the beams 308 corresponding to the upper leg and trunk of the patient 304 is greater at the second time than at the first time, indicating that the head and torso of the patient 304 is less elevated at the second time than at the first time. Furthermore, the position of the hip of the patient 304 has shifted away from the corner of the bend in the support structure 305. These factors indicate that the patient 304 has slid down in the support structure 305 between the first time and the second time. Sliding is associated with a heightened pressure injury risk.

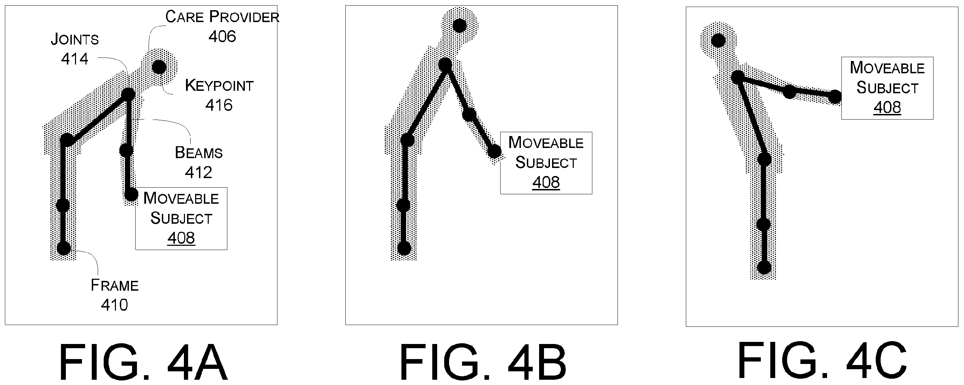

FIGS. 4A to 4C illustrate images 400, 402, and 404 of a care provider 406 lifting a moveable subject 408. In some implementations, the images 400, 402, and 404 are captured by one or more cameras, such as the camera(s) 102 described above with reference to FIG. 1. The care provider 406, for example, is the care provider 106 described above with reference to FIG. 1. The images 400, 402, and 404 may be captured from a camera positioned to the side of the care provider 406 as the care provider 406 is moving the moveable subject 408 (e.g., the movable subject 112 described above with reference to FIG. 1).

FIGS. 4A to 4C illustrate the care provider 408 lifting the moveable subject 408 over time. FIG. 4A illustrates a first time, FIG. 4B illustrates a second time, and FIG. 4C illustrates a third time. As shown, an object depicting the care provider 406 is overlaid with a frame 410 of the care provider 406. The frame 410 includes multiple beams 412 and multiple joints 414. The frame 410 is overlaid over at least a portion of the skeleton of the care provider 406. The beams 412 may represent bones in the skeleton. The joints 414 represent flexible connection points between the bones of the skeleton, such as musculoskeletal joints or sockets. In addition, the frame 410 includes a keypoint 416 representing the head and/or face of the care provider 406. The frame 410 may be generated based on an image of the care provider 404 using a technique such as OpenPose.

By tracking the moveable subject 408 in the images 400, 402, and 404, a system may conclude that the moveable subject 408 is being lifted. In addition, by tracking the object of the care provider 406 over time, the system may conclude that the care provider 406 is lifting the moveable subject 408. In various implementations, the pose of the frame 408 of the care provider 406 can be analyzed in the images 400, 402, and 404 to determine whether the care provider 406 is using a safe lifting posture. If the frame 408 indicates that the care provider 406 has an unsafe lifting posture (e.g., the care provider 406 is rounding their back during the lift), then the system may determine that the care provider 406 is at a heightened risk for developing a lifting injury. For instance, given the distance between the torso of the care provider 406 and the moveable subject 408 in the second image 402, the care provider 406 may be exerting an unsafe force on their back and shoulders during the lift. If the moveable subject 408 was closer to the torso of the care provider 406, then the risk of the lifting injury may be decreased.

In some cases, the system further infers the weight of the moveable subject 408 based on the images 400, 402, and 404. For example, the third image 404 indicates that the torso of the care provider 406 is out of alignment with the legs of the care provider 406, as indicated by the hip angle of the frame 410. In order to maintain the misaligned posture in the third image 404, a system may infer that the body of the care provider 406 is counterbalanced by the weight of the moveable subject 408. For instance, if the weight of the care provider 406 is known, the eight of the moveable subject 408 may be inferred based on the posture of the care provider 406 and statics principles.

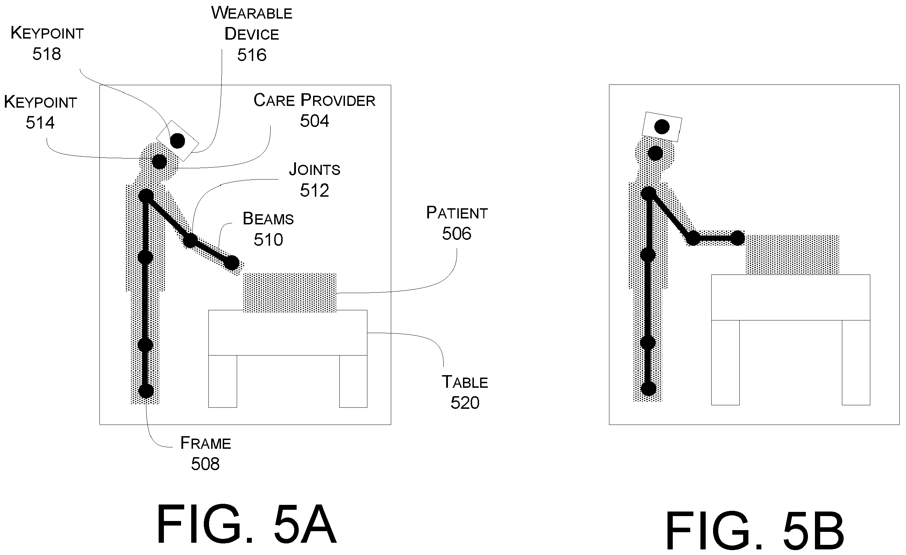

FIGS. 5A and 5B illustrate images 500 and 502 of a care provider 504 that is at risk for developing a posture-based injury. In some implementations, the images 500 and 502 are captured by one or more cameras, such as the camera(s) 102 described above with reference to FIG. 1. The care provider 504, for example, is the care provider 106 described above with reference to FIG. 1. The images 500 and 502 may be captured from a camera positioned to the side of the care provider 504 as the care provider 504 is performing a procedure on a patient 506 (e.g., the patient 104 described above with reference to FIG. 1).

As shown in both images 500 and 502, an object depicting the care provider 504 is overlaid with a frame 508 of the care provider 504. The frame 508 includes multiple beams 510 and multiple joints 512. The frame 508 is overlaid over at least a portion of the skeleton of the care provider 504. The beams 510 may represent bones in the skeleton. The joints 512 represent flexible connection points between the bones of the skeleton, such as musculoskeletal joints or sockets. In addition, the frame 508 includes a keypoint 514 representing the head and/or face of the care provider 504. The frame 508 may be generated based on an image of the care provider 504 using a technique such as OpenPose.

In addition, the care provider 504 is wearing a wearable device 516 associated with a keypoint 518. For instance, the wearable device 516 is a surgical headlight device, smart glasses, or a set of loupes worn on the head of the care provider 504. In various cases, the care provider 504 can develop head, neck, and back injuries based on the posture of the care provider's 504 head over time and the weight of the wearable device 516. Thus, a system analyzing the images 500 and 502 may determine the care provider's 504 risk for developing the posture-based injury.

FIG. 5A illustrates a first image 500 of the care provider 504 adopting a risky posture. As shown, the head of the care provider 504 is significantly angled with respect to the torso of the care provider 504, which can cause strain on the neck of the care provider 504. Furthermore, the forearm of the care provider 504 is angled down toward the patient 506, which can cause arm and wrist injuries over time. In the first image 500, a table 520 supporting the patient 506 is at a relatively low height. In some cases, the system may determine, based on the first mage 500, that the care provider 504 has a relatively high risk for developing a posture-based injury. The system may output a report and/or alert based on the risk. For example, the report and/or alert may instruct the care provider 504 to raise the table 520 to improve the posture of the care provider 504. In some cases, the report and/or alert is transmitted and output by the wearable device 516. Other examples of a risky posture include at least one of the care provider 504 having a rounded back, a torso of the care provider 504 being flexed, the care provider 504 having a stooped posture, a hand of the care provider 504 being extended greater than a threshold distance from the torso of the care provider 504, a hand of the care provider 504 being disposed above a shoulder of the care provider 504, a limb or joint (e.g., a wrist) of the care provider 504 being bent at greater than a first threshold angle, or a neck of the care provider 504 being bent at greater than a second threshold angle.

FIG. 5B illustrates a second image 502 of the care provider 504 adopting a relatively ergonomic posture. The table 520 is at a higher position in the second image 502 than in the first image 504, which enables the improvement in posture of the care provider 504. In particular, the head of the care provider 504 is more aligned with the torso of the care provider 504 in the second image 502. Further, the forearm of the care provider 504 is parallel to the surface of the table 520, which supports better alignment in the wrist of the care provider 504 as they are performing the procedure on the patient 506. Based on these factors, the system may determine that the care provider 504 has a relatively low risk for developing a posture-based injury based on the second image 502.

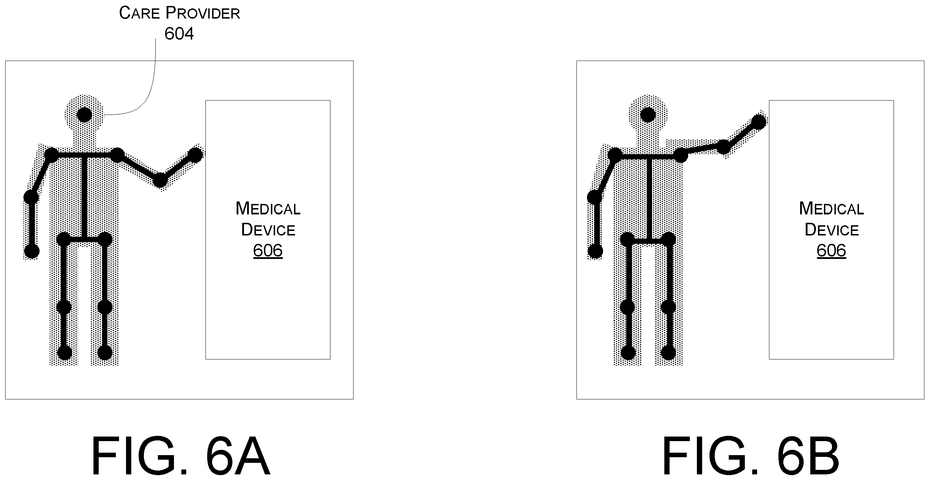

FIGS. 6A and 6B illustrate images 600 and 602 of a care provider 604 that is at risk for developing a repetitive-use injury. In some implementations, the images 600 and 602 are captured by one or more cameras, such as the camera(s) 102 described above with reference to FIG. 1. The care provider 604, for example, is the care provider 106 described above with reference to FIG. 1. The images 600 and 602 may be captured from a camera positioned to the side of the care provider 604 as the care provider 604 is operating a medical device 606 (e.g., the medical device 114 described above with reference to FIG. 1).

As shown in both images 600 and 602, an object depicting the care provider 604 is overlaid with a frame 608 of the care provider 604. The frame 608 includes multiple beams 610 and multiple joints 612. The frame 608 is overlaid over at least a portion of the skeleton of the care provider 604. The beams 610 may represent bones in the skeleton. The joints 612 represent flexible connection points between the bones of the skeleton, such as musculoskeletal joints or sockets. In addition, the frame 608 includes a keypoint 614 representing the head and/or face of the care provider 604. The frame 608 may be generated based on an image of the care provider 604 using a technique such as OpenPose.

FIG. 6A illustrates the care provider 604 at a first time and a first pose. FIG. 6B illustrates the care provider 604 at a second time and a second pose. In various implementations, a system may determine the risk that the care provider 604 will develop a repetitive-use injury by counting a number of times that the care provider 604 moves between the first pose and the second pose over a time interval. As the number of times that the care provider 604 repeats the motion increases, so does the risk for the care provider 604 developing the repetitive-use injury.

Furthermore, the type of repeated motion and/or poses made by the care provider 604 also impacts the risk of the repetitive-use injury. The risk for the repetitive-use injury increases if the repetitive poses include a pose in which the care provider 604 is raising their hand above their head, as shown in FIG. 6B. Other factors that impact the risk include whether the motion is weight-bearing (e.g., the care provider 604 is carrying a weight-bearing object while performing the motion), which can further increase the risk and/or whether the care provider 604 has adopted a risky posture (e.g., which can increase the risk of developing the repetitive-use injury).

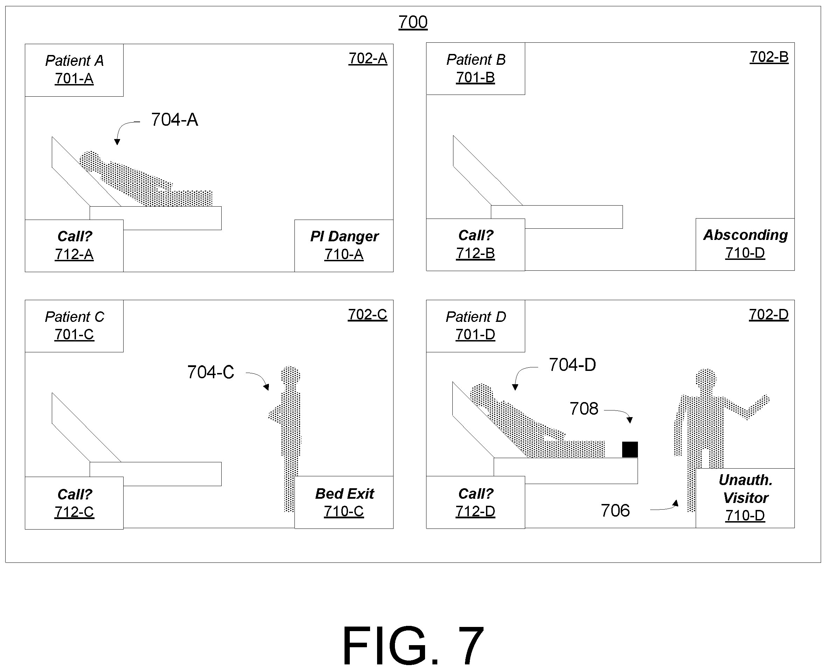

FIG. 7 illustrates an example of a user interface 700 that is output by a monitoring terminal. For example, the monitoring terminal 146 described above with reference to FIG. 1 may be configured to output the user interface 700. In various examples, a risk tracking system (e.g., the risk tracking system 126 described above with reference to FIG. 1) is configured to generate the user interface 700 and cause the monitoring terminal to output the user interface 700 to a monitoring user.

The user interface 700 illustrated in FIG. 7 includes four video windows 702-A to 702-D that are used to monitor different patient rooms, simultaneously. The four video windows 702-A to 702-D display images and/or videos in real-time or substantially real-time. The four windows 702-A to 702-D are respectively include labels 701-A to 701-D that identify the patient rooms being monitored. For example, the labels 701-A to 701-D indicate hospital identifiers or names associated with the patient rooms.

The first video window 702-A illustrates a first patient 704-A resting in a support structure. By analyzing images in the video displayed in the first video window 702-A, the risk tracking system may identify that the first patient 704-A has a greater-than-threshold risk of developing a pressure injury (e.g., within the next day). For instance, the first patient 704-A has slid down in the support structure, thereby creating a shear force on the back of the first patient 704-A that increases their risk for a pressure injury, the first patient 704-A has been moving less than a threshold frequency, or some other event increasing the pressure injury risk has occurred.

The second video window 702-B illustrates a second patient room. However, the second patient is not depicted in the second patient room. Accordingly, the risk tracking system may determine that the second patient has a greater-than-threshold risk of having absconded.

The third video window 702-C illustrates a third patient room. The third video window 702-C depicts a third patient 704-C who is standing up from a support structure in the third patient room. Accordingly, the risk tracking system may analyze the images to determine that the third patient 704-C has a greater-than-threshold risk of having made an unauthorized bed exit.

The fourth video window 702-D illustrates a fourth patient room. The fourth video window 702-D depicts a fourth patient 702-D who is resting on a support structure. However, the fourth video window 702-D also depicts a visitor 706 in the fourth patient room. In some cases, the risk tracking system may determine that the visitor 706 is an unauthorized visitor, and thus infer that the fourth patient 702-D has a greater-than-threshold risk of having an unauthorized visitor. Further, the fourth video window 702-D depicts a food item 708. Based on tracking the food item 708 in the images, the risk tracking system may determine that the fourth patient 704-D has a greater-than-threshold risk of participating in unauthorized eating or drinking.

Based on any of the risks identified based on the images, the risk tracking system may generate the user interface 700 to emphasize and/or alert a viewer of dangers posed by the risks. In particular implementations, the video windows 702-A to 702-D are overlaid with pop-ups 710-A to 710-D that respectively alert the viewer about the respective greater-than-threshold risks associated with the monitored patients 704-A to 704-D. The user interface 700 may alternatively or additionally warn the viewer of the risks in other ways. For example, the risk tracking system may highlight or otherwise emphasize objects-of-interest (e.g., the food item 708) in the video windows 702-A to 702-D to draw attention to the objects-of-interest. In some cases, the risk tracking system highlights or otherwise emphasizes any of the video windows 702-A to 702-D that are associated with a greater-than-threshold risk of patient injury. Furthermore, the monitoring terminal may output other signals to alert and draw attention to one or more of the video windows 702-A to 702-D, such as vibration and/or audible signals (e.g., beeps or alarms).

The user interface 700 further includes call elements 712-A to 712-D respectively associated with the video windows 702-A to 702-D. The call elements 712-A to 712-D may be pop-ups overlaid on the video windows 702-A to 702-D, but implementations are not so limited. In various cases, the viewer may contact a care provider or security official by selecting any of the call elements 712-A to 712-D. For instance, upon selecting a first call element 712-A associated with the first video window 702-A, the monitoring terminal may transmit a text message or voice call to a computing device of care provider responsible for caring for the first patient 704-A in order to alert the care provider of the pressure injury risk of the first patient 704-A. In some cases, upon selecting a fourth call element 712-D associated with the fourth video window 702-D, the monitoring terminal may transmit a text message or voice call to a computing device of a security person responsible for maintaining security on the premises of the clinical setting, in order to alert the security person of the unauthorized visitor 706.

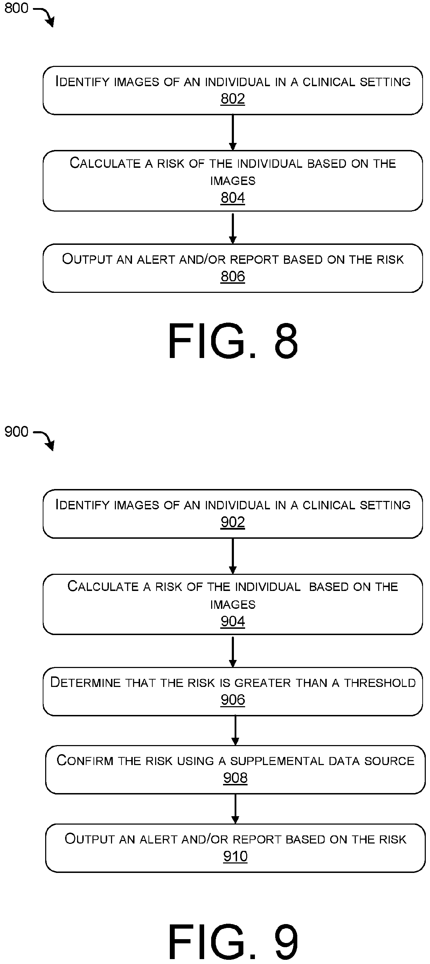

FIG. 8 illustrates an example process 800 for image-based analysis of the risk of an individual in a clinical setting. The process 800 may be performed by an entity, such as the risk tracking system 126, a computing device, or a processor.

At 802, the entity identifies images of an individual in a clinical setting. For example, the images are obtained by a camera in the clinical setting. The individual may be a patient, a care provider, or a visitor in the clinical setting. According to some implementations, the images depict a room or other space assigned to the patient. In some cases, the images depict an operating room, laboratory, office, or other space where the care provider is performing clinical duties. In some examples, the images are a video of the individual in the clinical setting.

At 804, the entity calculates a risk of the individual based on the images. According to various implementations, the entity detects, identifies, and tracks an object corresponding to the individual in the images. In some cases, the entity detects, identifies, and tracks other objects corresponding to other subjects depicted in the images, such as other individuals, a food item, a moveable subject, a medical device, or a support structure. The entity may calculate the risk of the individual based on this analysis.

In some cases, the entity calculates the risk that the patient has and/or will develop an injury or other clinically relevant problem. For example, the entity may analyze the images in order to calculate a pressure injury risk of the patient, a risk that the patient has participated and/or will participate in an unauthorized bed exit, a risk that the patient has and/or will aspirate, a risk that the patient has and/or will experience a fall, a risk that the patient has experienced a seizure, a risk that the patient has been visited by an unauthorized visitor, a risk that the patient is participating and/or will participate in unauthorized eating or drinking, a risk that the patient has absconded and/or will abscond, a risk that the patient has experienced and/or will experience a moment of wakefulness, a risk that the patient has self-harmed and/or will self-harm, or any combination thereof.

In some implementations, the entity calculates the risk that the care provider has and/or will develop an injury. For example, the entity may analyze the images in order to calculate a risk that the care provider has and/or will experience an act of violence against the care provider, a risk that the care provider will develop a repetitive use injury, a risk that the care provider will develop a posture-based injury, a risk that the care provider will develop a lifting injury, or any combination thereof.

The entity may rely on supplemental sources of data to calculate and/or confirm the risk to the patient and/or the care provider. For example, the entity may determine the risk to the patient based on data from the medical device, data from the support structure, data from an EMR of the patient, or data from a location tracking system. In some instances, the entity may determine the risk to the care provider based on data from the location tracking system, data from a wearable sensor, or data from a sensor affixed to and/or integrated with a moveable subject.

At 806, the entity outputs an alert and/or report based on the risk. In some cases, the entity transmits the alert and/or report to an external device, such as a wearable device, a clinical device, an administrator device, a security device, or a monitoring terminal. The external device may output the alert and/or report to a user. In various cases, the alert and/or report indicates the risk. In some cases, the alert and/or report includes an instruction for solving and/or reducing the risk to the patient or care provider. According to some implementations, the entity selectively outputs the alert upon determining that a risk exceeds a threshold.

FIG. 9 illustrates an example process 900 for confirming an image-based analysis of the risk of an individual in a clinical setting using supplemental data sources. The process 900 may be performed by an entity, such as the risk tracking system 126, a computing device, or a processor.

At 902, the entity identifies images of an individual in a clinical setting. For example, the images are obtained by a camera in the clinical setting. The individual may be a patient, a care provider, or a visitor in the clinical setting. According to some implementations, the images depict a room or other space assigned to the patient. In some cases, the images depict an operating room, laboratory, office, or other space where the care provider is performing clinical duties. In some examples, the images are a video of the individual in the clinical setting.

At 904, the entity calculates a risk of the individual based on the images. According to various implementations, the entity detects, identifies, and tracks an object corresponding to the individual in the images. In some cases, the entity detects, identifies, and tracks other objects corresponding to other subjects depicted in the images, such as other individuals, a food item, a moveable subject, a medical device, or a support structure. The entity may calculate the risk of the individual based on this analysis.

In some cases, the entity calculates the risk that the patient has and/or will develop an injury or other clinically relevant problem. For example, the entity may analyze the images in order to calculate a pressure injury risk of the patient, a risk that the patient has participated and/or will participate in an unauthorized bed exit, a risk that the patient has and/or will aspirate, a risk that the patient has and/or will experience a fall, a risk that the patient has experienced a seizure, a risk that the patient has been visited by an unauthorized visitor, a risk that the patient is participating and/or will participate in unauthorized eating or drinking, a risk that the patient has absconded and/or will abscond, a risk that the patient has experienced and/or will experience a moment of wakefulness, a risk that the patient has self-harmed and/or will self-harm, or any combination thereof.

In some implementations, the entity calculates the risk that the care provider has and/or will develop an injury. For example, the entity may analyze the images in order to calculate a risk that the care provider has and/or will experience an act of violence against the care provider, a risk that the care provider will develop a repetitive use injury, a risk that the care provider will develop a posture-based injury, a risk that the care provider will develop a lifting injury, or any combination thereof.

At 906, the entity determines that the risk is greater than a threshold. In some cases, the threshold is predetermined. In various cases, the threshold is user-adjusted, such as by an administrator of the clinical setting. For example, the entity may receive a signal indicative of the threshold from an administrator device.

At 908, the entity confirms the risk using a supplemental data source. For example, the entity may confirm the risk to the patient based on data from the medical device, data from the support structure, data from an EMR of the patient, or data from a location tracking system. In some instances, the entity may confirm the risk to the care provider based on data from the location tracking system, data from a wearable sensor, or data from a sensor affixed to and/or integrated with a moveable subject.

At 910, the entity outputs an alert and/or report based on the risk. In some cases, the entity transmits the alert and/or report to an external device, such as a wearable device, a clinical device, an administrator device, a security device, or a monitoring terminal. The external device may output the alert and/or report to a user. In various cases, the alert and/or report indicates the risk. In some cases, the alert and/or report includes an instruction for solving and/or reducing the risk to the patient or care provider. According to some implementations, the entity selectively outputs the alert upon determining that a risk exceeds a threshold.

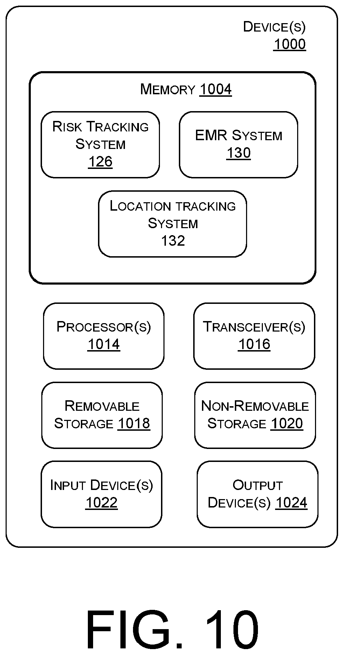

FIG. 10 illustrates at least one example device 1000 configured to enable and/or perform the some or all of the functionality discussed herein. Further, the device(s) 1000 can be implemented as one or more server computers 1002, a network element on a dedicated hardware, as a software instance running on a dedicated hardware, or as a virtualized function instantiated on an appropriate platform, such as a cloud infrastructure, and the like. It is to be understood in the context of this disclosure that the device(s) 1000 can be implemented as a single device or as a plurality of devices with components and data distributed among them.

As illustrated, the device(s) 1000 comprise a memory 1004. In various embodiments, the memory 1004 is volatile (including a component such as Random Access Memory (RAM)), non-volatile (including a component such as Read Only Memory (ROM), flash memory, etc.) or some combination of the two.

The memory 1004 may include various components, such as the risk tracking system 126, the EMR system 130, and the location tracking system 132. Any of the risk tracking system 126, the EMR system 130, and the location tracking system 132 can include methods, threads, processes, applications, or any other sort of executable instructions. The risk tracking system 126, the EMR system 130, and the location tracking system 132 and various other elements stored in the memory 1004 can also include files and databases.

The memory 1004 may include various instructions (e.g., instructions in the the risk tracking system 126, the EMR system 130, and the location tracking system 132), which can be executed by at least one processor 1014 to perform operations. In some embodiments, the processor(s) 1014 includes a Central Processing Unit (CPU), a Graphics Processing Unit (GPU), or both CPU and GPU, or other processing unit or component known in the art.

The device(s) 1000 can also include additional data storage devices (removable and/or non-removable) such as, for example, magnetic disks, optical disks, or tape. Such additional storage is illustrated in FIG. 10 by removable storage 1018 and non-removable storage 1020. Tangible computer-readable media can include volatile and nonvolatile, removable and non-removable media implemented in any method or technology for storage of information, such as computer readable instructions, data structures, program modules, or other data. The memory 1004, removable storage 1018, and non-removable storage 1020 are all examples of computer-readable storage media. Computer-readable storage media include, but are not limited to, RAM, ROM, EEPROM, flash memory or other memory technology, CD-ROM, Digital Versatile Discs (DVDs), Content-Addressable Memory (CAM), or other optical storage, magnetic cassettes, magnetic tape, magnetic disk storage or other magnetic storage devices, or any other medium which can be used to store the desired information and which can be accessed by the device(s) 1000. Any such tangible computer-readable media can be part of the device(s) 1000.

The device(s) 1000 also can include input device(s) 1022, such as a keypad, a cursor control, a touch-sensitive display, voice input device, etc., and output device(s) 1024 such as a display, speakers, printers, etc. These devices are well known in the art and need not be discussed at length here. In particular implementations, a user can provide input to the device(s) 500 via a user interface associated with the input device(s) 1022 and/or the output device(s) 1024.

As illustrated in FIG. 10, the device(s) 1000 can also include one or more wired or wireless transceiver(s) 1016. For example, the transceiver(s) 1016 can include a Network Interface Card (NIC), a network adapter, a LAN adapter, or a physical, virtual, or logical address to connect to the various base stations or networks contemplated herein, for example, or the various user devices and servers. To increase throughput when exchanging wireless data, the transceiver(s) 1016 can utilize Multiple-Input/Multiple-Output (MIMO) technology. The transceiver(s) 1016 can include any sort of wireless transceivers capable of engaging in wireless, Radio Frequency (RF) communication. The transceiver(s) 1016 can also include other wireless modems, such as a modem for engaging in Wi-Fi, WiMAX, Bluetooth, or infrared communication. In some implementations, the transceiver(s) 1016 can be used to communicate between various functions, components, modules, or the like, that are comprised in the device(s) 1000.

In some instances, one or more components may be referred to herein as "configured to," "configurable to," "operable/operative to," "adapted/adaptable," "able to," "conformable/conformed to," etc. Those skilled in the art will recognize that such terms (e.g., "configured to") can generally encompass active-state components and/or inactive-state components and/or standby-state components, unless context requires otherwise.

As used herein, the term "based on" can be used synonymously with "based, at least in part, on" and "based at least partly on."

As used herein, the terms "comprises/comprising/comprised" and "includes/including/included," and their equivalents, can be used interchangeably. An apparatus, system, or method that "comprises A, B, and C" includes A, B, and C, but also can include other components (e.g., D) as well. That is, the apparatus, system, or method is not limited to components A, B, and C.

Embodiments of the invention can be described with reference to the following numbered clauses, with preferred features laid out in the dependent clauses:
1. A system including: a camera configured to capture images of a patient disposed on a support structure; a load cell integrated into the support structure and configured to determine a weight of the patient on the support structure; a processor communicatively coupled to the camera and the load cell; memory communicatively coupled to the processor and storing instructions that, when executed by the processor, cause the processor to perform operations including: tracking, based on the images of the patient, a position of the patient over time; determining a risk of the patient for developing a pressure injury based on the position of the patient over time and the weight of the patient; determining that the risk of the patient for developing the pressure injury is greater than a threshold; generating a report based on determining that the risk of the patient developing for the pressure injury is greater than the threshold; and outputting the report.
2. The system of clause 1, the threshold being a first threshold, the system further including: a transceiver communicatively coupled to the processor and configured to receive, from a medical device, data indicating a vital sign of the patient, the vital sign including a heart rate, a respiration rate, or a blood oxygenation of the patient, wherein identifying the risk of the patient for developing the pressure includes determining whether the vital sign exceeds a second threshold or is lower than a second threshold.
3. The system of clause 1 or 2, further including: a transceiver communicatively coupled to the processor and configured to receive, from an electronic medical record (EMR) system, at least a portion of an EMR of the patient that includes at least one of a medication prescribed to the patient, an incontinence management device prescribed to the patient, a diagnosis of the patient, a demographic of the patient, a mobility level of the patient, a medical device used in the care of the patient, a nutritional intake of the patient, or a previous pressure injury experienced by the patient, wherein identifying the risk of the patient developing the pressure injury is further based on the at least the portion of the EMR of the patient.
4. The system one of clauses 1 to 3, the threshold being a first threshold, the system further including: a moisture sensor configured to detect moisture disposed on the support structure; and a temperature sensor configured to detect a temperature of the patient on the support structure, wherein identifying the risk of the patient developing the pressure injury includes: increasing the risk based on the moisture disposed on the support structure; or increasing the risk based on determining that the temperature of the support structure exceeds a second threshold or is less than a third threshold.
5. The system of one of clauses 1 to 4, the threshold being a first threshold, wherein the operations further include: identifying, based on the images and the weight, a bony protrusion or a weight of the patient, and identifying the risk of the patient developing the pressure injury includes: increasing the risk based on the bony protrusion; or increasing the risk based on determining that the weight of the patient exceeds a second threshold or is lower than a third threshold.
6. The system of one of clauses 1 to 5, wherein: tracking the position of the patient in the images includes determining a pose of the patient in the images; and identifying the risk of the patient for developing the pressure injury is based on the pose of the patient and includes: increasing the risk based on determining that a first limb of the patient is in contact with a second limb of the patient; increasing the risk based on determining that the patient has slid down the support structure; or increasing the risk based on determining that at least one foot of the patient is angled below a threshold level.
7. The system of one of clauses 1 to 6, wherein: the operations further include detecting, based on the images, an object or bedding fold disposed between the patient and the support structure, and identifying the risk of the patient developing the pressure injury includes increasing the risk based on detecting the object or the bedding fold disposed between the patient and the support structure.
8. The system of one of clauses 1 to 7, wherein tracking the portion of a patient over time includes identifying at least one of an amount of movement of the patient or a number of times that the patient has moved over time.
9. The system of one of clauses 1 to 8, wherein: the operations further include determining, based on the images, a frequency that at least one care provider changes bedding or incontinence pads of the patient, and the risk of the patient for developing the pressure injury is correlated to the frequency that the at least one care provider changes the bedding or the incontinence pads of the patient.
10. The system of one of clauses 1 to 9, wherein: the operations further include: determining, based on the images, a shear force between skin of the patient and the support structure, and the risk of the patient for developing the pressure injury is positively correlated to the shear force.
11. A system, including: a camera configured to capture images of a care provider over time; a processor communicatively coupled to the camera; memory communicatively coupled to the processor and storing instructions that, when executed by the processor, causes processor to perform operations including: determining, based on the images, a pose of the care provider over time; determining, based on the pose of the care provider over time, a risk that the care provider will develop an injury; determining that the risk exceeds a threshold; and outputting a report based on determining that the risk exceeds the threshold.
12. The system of clause 11, wherein: determining the risk that the care provider will develop the injury includes: detecting, based on the images, the care provider lifting a subject; identifying the weight of the subject, and determining the risk that the care provider will develop a lifting injury based on the pose of the care provider when the care provider is lifting the subject and the weight of the subject, the risk that the care provider will develop the lifting injury being positively correlated to the weight of the subject.
13. The system of clause 12, wherein identifying the weight of the subject includes analyzing the pose of the care provider holding the subject.
14. The system of clause 12 or 13, wherein identifying the weight of the subject includes: determining, based on the images, an identity of the subject; accessing an entry of a database based on the identity of the subject; and determining the weight based on the entry.
15. The system of one of clauses 11 to 14, wherein determining the risk that the care provider will develop the injury includes: determining, based on the pose of the care provider, that the pose of the care provider includes a risky posture, the risky posture including at least one of the care provider having a rounded back, a torso of the care provider being flexed, the care provider having a stooped posture, a hand of the care provider being extended greater than a threshold distance from the torso of the care provider, a hand of the care provider being disposed above a shoulder of the care provider, a wrist of the care provider being bent at greater than a first threshold angle, or a neck of the care provider being bent at greater than a second threshold angle; determining a time that the care provider is in the risky posture; and determining a risk of a posture-based injury based on the time that the care provider is in the risky posture, the risk of the posture-based injury being positively correlated to the time that the care provider is in the risky posture.
16. The system of one of clauses 11 to 15, wherein determining the risk that the care provider will develop the injury includes: determining, based on the pose of the care provider over time, a number of times that the care provider repeats a movement; and determining a risk of a repetitive-use injury based on the number of times that the care provider repeats the movement, the risk of the repetitive-use injury being positively correlated to the number of times that the care provider repeats the movement.
17. The system of clause 16, wherein the movement includes a risky posture, the risky posture including the care provider having a rounded back, a hand of the care provider being disposed above a shoulder of the care provider, a wrist of the care provider being bent at greater than a first threshold angle, or a neck of the care provider being bent at greater than a second threshold angle.
18. The system of one of clauses 11 to 17, further including: a transceiver configured to receive, from an external device, a signal indicating a parameter of the care provider, wherein determining the risk that the care provider will develop the injury is further based on the parameter of the care provider.
19. The system of clause 18, wherein: the external device includes a wearable device worn by the care provider; and the parameter includes an acceleration of at least a portion of the care provider or a heart rate of the care provider.
20. The system of one of clauses 11 to 19, further including: a transceiver configured to transmit, to an external device, a signal based on the report, wherein the external device includes a computing device associated with the care provider or a computing device associated with an administrator.
21. The system of clause 20, wherein the external device includes a wearable device associated with the care provider.
22. The system of one of clauses 11 to 21, wherein the report includes a recommendation to use an assistive device.
23. The system of one of clauses 11 to 22, further including: an assistive device located in a room of a clinical setting, the assistive device being configured to output a reminder notification, wherein the operations further include: determining that the care provider is located in the room; and based on determining that the risk exceeds the threshold and determining that the care provider is located in the room, causing the assistive device to output the reminder notification to the care provider.
24. A system, including: a first camera configured to detect first images of a first room in a clinical setting; a second camera configured to detect second images of a second room in the clinical setting; a display configured to output the first images and the second images; a processor communicatively coupled to the first camera and the second camera; and memory communicatively coupled to the processor storing instructions that, when executed by the processor, cause the processor to perform operations including: tracking a first patient in the first images; detecting a first event based on at least one of a proximity of a first object to the first patient in the first images, a motion of the first patient in the first images, or a pose of the first patient in the first images; determining, based on the first event, a risk that the first patient has experienced or will experience an adverse event; determining that the first probability exceeds a first threshold; tracking a second patient in the second images; detecting a second event based on at least one of a proximity of a second object to the second patient in the second images, a motion of the second patient in the second images, or a posture of the second patient in the second images; determining, based on the second event, a risk that the second patient has experienced a second adverse event; determining that the second probability is less than a second threshold; and based on determining that the first probability exceeds the first threshold and that the second probability is less than the second threshold, causing the display to visually emphasize the first images over the second images.
25. The system of clause 24, wherein: the first images include a video of the first patient in the first room, and the second images include a video of the second patient in the second room.
26. The system of clause 24 or 25, wherein the first object includes at least one of a care provider, a visitor of the first patient, a support structure, a sheet disposed on the support structure, a medical device, a vital sign monitor, a vital sign sensor, a food item, a food tray, or a drink.
27. The system of one of clauses 24 to 26, wherein the first adverse event includes at least one of a condition associated with a pressure injury, an unauthorized bed exit, aspiration, a fall, a seizure, a visit form an unauthorized visitor, unauthorized eating or drinking, absconding, wakefulness, or self-harm.
28. The system of one of clauses 23 to 27, wherein determining the first probability that the first patient has experienced the first adverse event is further based on at least one of an electronic medical record (EMR) of the first patient, a previous event experienced by the first patient, a pressure detected by at least one pressure sensor integrated in support structure of the first patient, moisture detected by at least one moisture sensor integrated in the support structure, a temperature detected by at least one temperature sensor integrated in the support structure, a sound detected by a microphone, a vital sign of the first patient, or the presence of a care giver in the first room.
29. The system of one of clauses 23 to 28, wherein causing the display to visually emphasize the first images over the second images includes at least one of: causing the display to output the first images in a first window and causing the display to output the second images in the second window, wherein the first window is larger than the second window; causing the display to highlight at least one element of the first images in the first window; or causing the display to output the first images with an overlay emphasizing the first images and causing the display to output the second images without the overlay.
30. A method, including: identifying images of an individual in a clinical setting, the individual including a patient or a care provider; calculating a risk of the individual experiencing an adverse event based on the images; and outputting a report based on the risk.
31. The method of clause 30, wherein the adverse event includes an injury.
32. The method of clause 30 or 31, wherein the individual is the patient and the adverse event includes at least one of a pressure injury, a bed exit, aspiration, a fall, a seizure, a visit from an unauthorized visitor, unauthorized eating or drinking, absconding, wakefulness, or self-harm.
33. The method of clause 32, further including: confirming or modifying the risk based on at least one of a vital sign of the patient detected by a medical device, a parameter detected by a support structure of the patient, or an electronic medical record (EMR) of the patient.
34. The method of one of clauses 30 to 33, wherein the individual is the care provider and the adverse event includes at least one of an act of violence, a lifting injury, a posture-based injury, or a repetitive use injury.
35. The method of clause 34, further including: confirming the risk based on at least one of a location of the care provider detected by a location tracking system, a parameter detected by a wearable sensor of the care provider, or a parameter detected by a sensor of a moveable subject that is moved by the care provider.
36. The method of one of clauses 30 to 35, wherein the report includes at least one of an indication of the risk or an instruction for reducing the risk.
37. The method of one of clauses 30 to 36, wherein outputting the report includes transmitting the report to an external device.
38. The method of one of clauses 30 to 37, further including: determining that the risk exceeds a threshold.
39. A computing device, including: a processor; and memory communicatively coupled to the processor and storing instructions that, when executed by the processor, cause the processor to perform operations including the method of one of clauses 30 to 38.

## Claims

1. A system comprising:
a camera configured to capture images of a patient disposed on a support structure;
a load cell integrated into the support structure and configured to determine a weight of the patient on the support structure;
a processor communicatively coupled to the camera and the load cell;
memory communicatively coupled to the processor and storing instructions that, when executed by the processor, cause the processor to perform operations comprising:
tracking, based on the images of the patient, a position of the patient over time;
determining a risk of the patient for developing a pressure injury based on the position of the patient over time and the weight of the patient;
determining that the risk of the patient for developing the pressure injury is greater than a threshold;
generating a report based on determining that the risk of the patient developing for the pressure injury is greater than the threshold; and
outputting the report.

2. The system of claim 1, the threshold being a first threshold, the system further comprising:
a transceiver communicatively coupled to the processor and configured to receive, from a medical device, data indicating a vital sign of the patient, the vital sign comprising a heart rate, a respiration rate, or a blood oxygenation of the patient,
wherein identifying the risk of the patient for developing the pressure comprises determining whether the vital sign exceeds a second threshold or is lower than a second threshold.

3. The system of either claim 1 or claim 2, further comprising:
a transceiver communicatively coupled to the processor and configured to receive, from an electronic medical record (EMR) system, at least a portion of an EMR of the patient that comprises at least one of a medication prescribed to the patient, an incontinence management device prescribed to the patient, a diagnosis of the patient, a demographic of the patient, a mobility level of the patient, a medical device used in the care of the patient, a nutritional intake of the patient, or a previous pressure injury experienced by the patient,
wherein identifying the risk of the patient developing the pressure injury is further based on the at least the portion of the EMR of the patient.

4. The system of any preceding claim, the threshold being a first threshold, the system further comprising:
a moisture sensor configured to detect moisture disposed on the support structure; and
a temperature sensor configured to detect a temperature of the patient on the support structure,
wherein identifying the risk of the patient developing the pressure injury comprises:
increasing the risk based on the moisture disposed on the support structure; or
increasing the risk based on determining that the temperature of the support structure exceeds a second threshold or is less than a third threshold.

5. The system of any preceding claim, the threshold being a first threshold, wherein the operations further comprise:
identifying, based on the images and the weight, a bony protrusion or a weight of the patient, and
identifying the risk of the patient developing the pressure injury comprises:
increasing the risk based on the bony protrusion; or
increasing the risk based on determining that the weight of the patient exceeds a second threshold or is lower than a third threshold.

6. The system of any preceding claim, wherein:
tracking the position of the patient in the images comprises determining a pose of the patient in the images; and
identifying the risk of the patient for developing the pressure injury is based on the pose of the patient and comprises:
increasing the risk based on determining that a first limb of the patient is in contact with a second limb of the patient;
increasing the risk based on determining that the patient has slid down the support structure; or
increasing the risk based on determining that at least one foot of the patient is angled below a threshold level.

7. The system of any preceding claim, wherein:
the operations further comprise detecting, based on the images, an object or bedding fold disposed between the patient and the support structure, and
identifying the risk of the patient developing the pressure injury comprises increasing the risk based on detecting the object or the bedding fold disposed between the patient and the support structure.

8. The system of any preceding claim, wherein tracking the portion of a patient over time comprises identifying at least one of an amount of movement of the patient or a number of times that the patient has moved over time.

9. The system of any preceding claim, wherein:
the operations further comprise determining, based on the images, a frequency that at least one care provider changes bedding or incontinence pads of the patient, and
the risk of the patient for developing the pressure injury is correlated to the frequency that the at least one care provider changes the bedding or the incontinence pads of the patient.

10. The system of any preceding claim, wherein:
the operations further comprise:
determining, based on the images, a shear force between skin of the patient and the support structure, and
the risk of the patient for developing the pressure injury is positively correlated to the shear force.

11. A system, comprising:
a camera configured to capture images of a care provider over time;
a processor communicatively coupled to the camera;
memory communicatively coupled to the processor and storing instructions that, when executed by the processor, causes processor to perform operations comprising:
determining, based on the images, a pose of the care provider over time;
determining, based on the pose of the care provider over time, a risk that the care provider will develop an injury;
determining that the risk exceeds a threshold; and
outputting a report based on determining that the risk exceeds the threshold.

12. The system of claim 11, wherein:
determining the risk that the care provider will develop the injury comprises:
detecting, based on the images, the care provider lifting a subject;
identifying the weight of the subject, and
determining the risk that the care provider will develop a lifting injury based on the pose of the care provider when the care provider is lifting the subject and the weight of the subject, the risk that the care provider will develop the lifting injury being positively correlated to the weight of the subject.

13. The system of claim 12, wherein identifying the weight of the subject comprises analyzing the pose of the care provider holding the subject.

14. The system of either claim 12 or claim 13, wherein identifying the weight of the subject comprises:
determining, based on the images, an identity of the subject;
accessing an entry of a database based on the identity of the subject; and
determining the weight based on the entry.

15. The system of any one of claims 11 to 14, wherein determining the risk that the care provider will develop the injury comprises:
determining, based on the pose of the care provider, that the pose of the care provider comprises a risky posture, the risky posture comprising at least one of the care provider having a rounded back, a torso of the care provider being flexed, the care provider having a stooped posture, a hand of the care provider being extended greater than a threshold distance from the torso of the care provider, a hand of the care provider being disposed above a shoulder of the care provider, a wrist of the care provider being bent at greater than a first threshold angle, or a neck of the care provider being bent at greater than a second threshold angle;
determining a time that the care provider is in the risky posture; and
determining a risk of a posture-based injury based on the time that the care provider is in the risky posture, the risk of the posture-based injury being positively correlated to the time that the care provider is in the risky posture.
